# EUROPEAN PATENT SPECIFICATION

(11) **EP 4 087 516 B1**
(45) Date of publication and mention of the grant of the patent: **10.04.2024**
(21) Application number: 21749334.5
(22) Date of filing: 27.07.2021
(51) Int. Cl.: A61B 34/30, A61B 34/32, A61B 34/00, A61F 9/007, A61B 90/00, A61B 90/50, A61B 34/20

(54) **ROBOTIC SYSTEM FOR MICROSURGICAL PROCEDURES**
ROBOTERSYSTEM FÜR MIKROCHIRURGISCHE EINGRIFFE
SYSTÈME ROBOTIQUE POUR PROCÉDURES MICROCHIRURGICALES

(30) Priority: 28.07.2020 US 202063057391 P; 05.10.2020 US 202063087408 P
(43) Date of publication of application: 16.11.2022
(73) Proprietor: Forsight Robotics Ltd., 2069205 Yokneam Illit (IL)
(72) Inventor: GLOZMAN, Daniel, 4034723 Kfar Yona (IL); ARNOLD, Ofer, 2012900 Ma'ale Zvia (IL)
(74) Representative: Evens, Paul Jonathan
(86) International application number: PCT/IB2021/056784
(87) International publication number: WO 2022/023962

(56) References cited:
- WO-A1-2019/183236
- WO-A1-2019/222228
- WO-A1-2020/070501
- WO-A1-2020/154012
- JP-A- 2019 530 517
- US-A- 5 410 638
- US-A- 5 828 197
- US-A1- 2003 125 716
- US-A1- 2007 299 427
- US-A1- 2010 331 858
- US-A1- 2013 035 537
- US-A1- 2013 131 867
- US-A1- 2014 364 870
- US-A1- 2016 346 060
- US-A1- 2017 252 208
- US-A1- 2018 104 013
- US-A1- 2019 314 529
- US-A1- 2019 343 594
- US-A1- 2020 222 124
- US-B1- 9 383 832
- CHEN CHENG-WEI ET AL: "Semiautomated optical coherence tomography-guided robotic surgery for porcine lens removal", JOURNAL CATARACT AND REFRACTIVE SURGERY, SURGERY, FAIRFAX, VA, US, vol. 45, no. 11, 1 November 2019 (2019-11-01), pages 1665-1669, XP085888767, ISSN: 0886-3350, DOI: 10.1016/J.JCRS.2019.06.020

## Description

### FIELD OF EMBODIMENTS OF THE INVENTION

Some applications of the present invention generally relate to medical apparatus. Specifically, some applications of the present invention relate to apparatus for performing microsurgical procedures in a robotic manner.

### BACKGROUND

Cataract surgery involves the removal of the natural lens of the eye that has developed an opacification (known as a cataract), and its replacement with an intraocular lens. Such surgery typically involves a number of standard steps, which are performed sequentially.

In an initial step, the patient's face around the eye is disinfected (typically, with iodine solution), and their face is covered by a sterile drape, such that only the eye is exposed. When the disinfection and draping has been completed, the eye is anesthetized, typically using a local anesthetic, which is administered in the form of liquid eye drops. The eyeball is then exposed, using an eyelid speculum that holds the upper and lower eyelids open. One or more incisions (and typically two or three incisions) are made in the cornea of the eye. The incision(s) are typically made using a specialized blade, that is called a keratome blade. At this stage, lidocaine is typically injected into the anterior chamber of the eye, in order to further anesthetize the eye. Following this step, a viscoelastic injection is applied via the corneal incision(s). The viscoelastic injection is performed in order to stabilize the anterior chamber and to help maintain eye pressure during the remainder of the procedure, and also in order to distend the lens capsule.

In a subsequent stage, known as capsulorhexis, a part of the anterior lens capsule is removed. Various enhanced techniques have been developed for performing capsulorhexis, such as laser-assisted capsulorhexis, zepto-rhexis (which utilizes precision nano-pulse technology), and marker-assisted capsulorhexis (in which the cornea is marked using a predefined marker, in order to indicate the desired size for the capsule opening).

Subsequently, it is common for a fluid wave to be injected via the corneal incision, in order to dissect the cataract's outer cortical layer, in a step known as hydrodissection. In a subsequent step, known as hydrodelineation, the outer softer epi-nucleus of the lens is separated from the inner firmer endo-nucleus by the injection of a fluid wave. In the next step, ultrasonic emulsification of the lens is performed, in a process known as phacoemulsification. The nucleus of the lens is broken initially using a chopper, following which the outer fragments of the lens are broken and removed, typically using an ultrasonic phacoemulsification probe. Further typically, a separate tool is used to perform suction during the phacoemulsification. When the phacoemulsification is complete, the remaining lens cortex (i.e., the outer layer of the lens) material is aspirated from the capsule. During the phacoemulsification and the aspiration, aspirated fluids are typically replaced with irrigation of a balanced salt solution, in order to maintain fluid pressure in the anterior chamber. In some cases, if deemed to be necessary, then the capsule is polished. Subsequently, the intraocular lens (IOL) is inserted into the capsule. The IOL is typically foldable and is inserted in a folded configuration, before unfolding inside the capsule. At this stage, the viscoelastic is removed, typically using the suction device that was previously used to aspirate fluids from the capsule. If necessary, the incision(s) is sealed by elevating the pressure inside the bulbus oculi (i.e., the globe of the eye), causing the internal tissue to be pressed against the external tissue of the incision, such as to force closed the incision.

US 2003/0125716 relates to a method and apparatus for performing minimally invasive cardiac procedures. US 2003/0125716 discloses apparatus for performing a procedure on a portion of a body of a patient using one or more tools, the apparatus comprising:
a robotic unit configured to move the tool through six degrees-of-freedom; and
a control component that comprises at least one control-component arm configured to be moved by a user, the control-component arm comprising: a control-component tool disposed at the end of the control-component arm, the control-component tool defining a tip; three joints; three rotary encoders, each of the three rotary encoders coupled to a respective one of the joints and configured to detect movement of the respective joint and to generate rotary-encoder data indicative of an XYZ location of the tip of the control-component tool, in response thereto; and an inertial measurement unit comprising at least one of sensor selected from the group consisting of: a three-axis accelerometer, a three-axis gyroscope, and a three-axis magnetometer, the inertial measurement unit being configured to generate inertial-measurement-unit data; and
a computer processor configured to: receive the rotary-encoder data and the inertial-measurement-unit data, based upon the rotary-encoder data determine the XYZ location of the tip of the control-component tool, and move the robotic unit.

WO 2020/154012, which forms part of the state of the art under Article 54(3) EPC only, relates to a wearable user interface device, including apparatus for performing a procedure on a portion of a body of a patient using one or more tools, the apparatus comprising:
a robotic unit configured to move the tool through six degrees-of-freedom; and
a control component that comprises at least one control-component arm configured to be moved by a user, the control-component arm comprising: a control-component tool disposed at the end of the control-component arm, the control-component tool defining a tip; three joints; three rotary encoders, each of the three rotary encoders coupled to a respective one of the joints and configured to detect movement of the respective joint and to generate rotary-encoder data indicative of an XYZ location of the tip of the control-component tool, in response thereto; and an inertial measurement unit comprising at least one of sensor selected from the group consisting of: a three-axis accelerometer, a three-axis gyroscope, and a three-axis magnetometer, the inertial measurement unit being configured to generate inertial-measurement-unit data; and
a computer processor configured to: receive the rotary-encoder data and the inertial-measurement-unit data, based upon the rotary-encoder data determine the XYZ location of the tip of the control-component tool, and move the robotic unit,
wherein: the inertial measurement unit is disposed on the control-component tool; the inertial-measurement-unit data generated by the inertial measurement unit is indicative of an orientation of the tip of control-component tool; and the computer processor is further configured to: determine the orientation of the tip of the control-component tool based upon the inertial-measurement-unit data, and move the robotic unit in response to the XYZ location and the orientation of the tip of the control-component tool determined by the computer processor.

### SUMMARY

In accordance with aspects of the present invention, there is provided apparatus for performing a procedure on a portion of a body of a patient using one or more tools, as defined in any one of appended independent claims 1, 3 and 12. Embodiments of the present invention are defined in appended claims dependent on any of independent claims 1, 3 and 12. Methods of using the apparatus are described to help fully understand the present invention, but do not form part of the present invention.

In accordance with some applications of the present disclosure, a robotic system is used for a microsurgical procedure, such as intraocular surgery. It is noted that intraocular surgery and, particularly cataract surgery, is characterized by the fact that there is relatively little variability in ocular anatomy and/or geometry from one patient to another. In addition, cataract surgery has a relatively standardized procedural flow, the typical sequence of steps being as noted hereinabove in the Background section. In view of the relative standard dimensions and sequence of steps that are associated with cataract surgery, for some applications of the present invention, a robotic system is initially trained to perform one or more steps of cataract surgery in an automated manner, based upon standard ranges of dimensions of respective portions of a human eye.

Typically, the robotic system includes first and second robotic units (which are configured to hold tools), in addition to an imaging system, a display, and a control component, via which a healthcare professional is able to control the robotic units. Typically, the robotic system includes one or more computer processors, via which components of the system and a user (e.g., a healthcare professional) operatively interact with each other.

For some applications, during a training stage, movement of the robotic units (and/or control of other aspects of the robotic system) is at least partially controlled by a user (e.g., a healthcare professional). For example, the user may receive images of the patient's eye and the robotic units, and/or tools disposed therein, via a display. Typically, such images are acquired by an imaging system. For some applications, the imaging system is a stereoscopic imaging device and the display is a stereoscopic display. Based on the received images, the user typically performs steps of the procedure. For some applications, the user provides commands to the robotic units via a control component (which is described in further detail hereinbelow). Typically, such commands include commands that control the position and/or orientation of tools that are disposed within the robotic units, and/or commands that control actions that are performed by the tools. For example, the commands may control a phacoemulsification tool (e.g., the operation mode and/or suction power of the phacoemulsification tool), and/or injector tools (e.g., which fluid (e.g., viscoelastic fluid, saline, etc.) should be injected, and/or at what flow rate). Alternatively or additionally, the user may input commands that control the imaging system (e.g., the zoom, focus, and/or x-y positioning of the imaging system). For some applications, the commands include controlling an IOL-manipulator tool, for example, such that the tool manipulates the IOL inside the eye for precise positioning of the IOL within the eye.

For some applications, after the initial training phase, the robotic system is used to perform one or more steps of cataract surgery on an eye of a given patient, in an at least partially automated manner. For some such applications, at least one image of the patient's eye is acquired using an imaging system. For example, the image may be acquired prior to or during one or more steps of cataract surgery being performed on the eye of the patient. Typically, the computer processor analyzes the image in order to determine precise dimensions of the patient's eye, and the steps of the cataract surgery are planned based upon the training and the determined dimensions of the eye. Typically, such steps involve the controlling the positions and orientation of tools via the robotic units, as well as controlling additional aspects of the procedure (such as controlling functions of the tools, and/or the functions and movements of the imaging system), as described hereinabove. Subsequent thereto, the computer processor typically drives the robotic units and/or additional components of the robotic system to perform the planned steps. For some applications, during the procedure, a healthcare professional is able to control movement of the robotic units and/or other aspects of the robotic system. For example, the computer processor may plan a step of the procedure and indicate the planned step to the healthcare professional. The healthcare professional may then override and/or refine the planned step of the procedure e.g., via the control component.

For some applications, any one of the following steps of a cataract procedure (or a portion thereof, or combination thereof) is performed in an automated or semi-automated manner using the techniques described herein: corneal incision (e.g., a bi-planar or tri-planar incision, typically using a keratome blade), viscoelastic injection (including needle insertion via one of the incisions and/or simultaneous injection and retraction of the needle), capsulorhexis, hydrodissection, hydrodelineation, phacoemulsification (including nuclear chopping, and/or ultrasonic emulsification using a phacoemulsification probe), irrigation, aspiration, implantation of an IOL (e.g., using a IOL injection system), viscoelastic removal, wound healing, and/or any combination thereof.

For some applications, the above-described steps of imaging the patient's eye, planning a procedural step, and moving the robotic units and/or controlling other aspects of the robotic system are performed in an iterative manner. Typically, throughout a given procedural step being performed, the patient's eye and the robotic units (and/or tools disposed therein) are continuously imaged in real time. In response to the real-time images, the position and orientation of the tool is updated to correspond with real-time movement of the patient's eye (as described in further detail hereinbelow). Typically, the position and orientation of the tool is updated by moving the tool via the robotic units. Alternatively or additionally, the computer processor is configured to detect when the eye is at a given position, and to time the performance of certain functions by the robotic units such that they are performed when the eye is at the given position.

Typically, the computer processor detects movement of the patient's eye in three dimensions, by analyzing images acquired by the imaging system (which as described hereinabove is typically a stereoscopic imaging system). For some applications, in response to the detected movement of the patient's eye, the computer processor drives the robotic unit to move the tool such that entry of the tool into the patient's eye remains via the incision point even as the patient's eye undergoes the movement in three dimensions. Typically, even as the patient's eye undergoes the movement in three dimensions, the computer processor drives the robotic unit to perform at least a portion of a procedure on the patient's eye by moving the tip of the tool in a desired manner with respect to the eye such as to perform the portion of the procedure, while entry of the tool into the patient's eye is maintained fixed at incision point. In this manner, the robotic unit acts to provide a dynamic remote center of motion that is located at the incision point, and about which motion of the tool is centered. Typically, the remote center of motion moves in coordination with movement of the eye. For some applications, the above-described steps are performed while the patient's eye is held substantially stationary by virtue of the tool being disposed within the eye. That is to say that, while the eye is held stationary, the computer processor drives the robotic unit to perform at least a portion of a procedure on the patient's eye by moving the tip of the tool in a desired manner with respect to the eye such as to perform the portion of the procedure, while entry of the tool into the patient's eye is maintained fixed at the incision point.

As described hereinabove, for some applications, the user provides commands to the robotic units via a control component. Typically, a control-component arm of the control component is configured to be moved by a user and to thereby control movement of an arm of a robotic unit. Typically, the control component includes first and second control-component arms that are configured to correspond to respective robotic units of the robotic system. For some applications, the control-component arms are configured to hold respective control-component tools therein (in order to replicate the arms of the robotic units). For applications in accordance with the present invention, each of the control-component arms includes three joints, and a respective rotary encoder coupled to each one of the three joints. The rotary encoders are configured to detect movement of the respective joints and to generate rotary-encoder data in response thereto. For applications in accordance with the present invention, the control-component arm additionally includes an inertial-measurement unit that includes a three-axis accelerometer, a three-axis gyroscope, and/or a three-axis magnetometer. The inertial-measurement unit typically generates inertial-measurement-unit data relating to a three-dimensional orientation of the control-component arm, in response to the control-component arm being moved. For some applications in accordance with the present invention, the computer processor receives the rotary-encoder data and the inertial-measurement-unit data. The computer processor determines the XYZ location of the tip of the control-component tool, based upon the rotary-encoder data, and determines the orientation of the tip of control-component tool (e.g., the 3 Euler angles of orientation, and/or another representation of orientation) based upon the inertial-measurement-unit data. Thus, based upon the combination of the rotary-encoder data and the inertial-measurement-unit data, the computer processor is configured to determine the XYZ location and orientation of the tip of the control-component tool.

For some applications, the computer processor drives the robotic unit such that the tip of the actual tool that is being used to perform the procedure tracks the movements of the tip of the control-component tool. Typically, incorporating an inertial-measurement unit to detect the three-dimensional orientation of the control-component arm allows the user to control movement of the robotic unit using a reduced number of sensors, relative to if rotary encoders were used to detect motion of the control-component arm in all six degrees-of-freedom. Further typically, reducing the number of rotary encoders that are used tends to reduce the overall complexity of the control component, since introducing additional rotary encoders would require additional wires to pass through rotating joints.

Although some applications of the present invention are described with reference to cataract surgery, the scope of the present application includes applying the apparatus described herein to other medical procedures, *mutatis mutandis.* In particular, the apparatus and methods described herein to other medical procedures may be applied to other microsurgical procedures, such as general surgery, orthopedic surgery, gynecological surgery, otolaryngology, neurosurgery, oral and maxillofacial surgery, plastic surgery, podiatric surgery, vascular surgery, and/or pediatric surgery that is performed using microsurgical techniques. For some such applications, the imaging system includes one or more microscopic imaging units.

It is noted that the scope of the present application includes applying the apparatus described herein to intraocular procedures, other than cataract surgery, *mutatis mutandis.* Such procedures may include collagen crosslinking, endothelial keratoplasty (e.g., DSEK, DMEK, and/or PDEK), DSO (descemet stripping without transplantation), laser assisted keratoplasty, keratoplasty, LASIK/PRK, SMILE, pterygium, ocular surface cancer treatment, secondary IOL placement (sutured, transconjunctival, etc.), iris repair, IOL reposition, IOL exchange, superficial keratectomy, Minimally Invasive Glaucoma Surgery (MIGS), limbal stem cell transplantation, astigmatic keratotomy, Limbal Relaxing Incisions (LRI), amniotic membrane transplantation (AMT), glaucoma surgery (e.g., trabs, tubes, minimally invasive glaucoma surgery), automated lamellar keratoplasty (ALK), anterior vitrectomy, and/or pars plana anterior vitrectomy.

There is therefore provided, in accordance with some applications of the present invention, apparatus for performing a procedure on a portion of a body of a patient using one or more tools, the apparatus including:
a robotic unit configured to move the tool through six degrees-of-freedom; and
a control component that includes at least one control-component arm configured to be moved by a user, the control-component arm including:
   a control-component tool disposed at the end of the control-component arm, the control-component tool defining a tip;
   three joints;
   three rotary encoders, each of the three rotary encoders coupled to a respective one of the joints and configured to detect movement of the respective joint and to generate rotary-encoder data indicative of an XYZ location of the tip of the control-component tool, in response thereto; and
   an inertial measurement unit including at least one of sensor selected from the group consisting of: a three-axis accelerometer, a three-axis gyroscope, and a three-axis magnetometer, the inertial measurement unit being configured to generate inertial-measurement-unit data; and
a computer processor configured to:
   receive the rotary-encoder data and the inertial-measurement-unit data,
   based upon the rotary-encoder data determine the XYZ location of the tip of the control-component tool, and
   move the robotic unit,
wherein:
   the inertial measurement unit is disposed on the control-component tool;
   the inertial-measurement-unit data generated by the inertial measurement unit is indicative of an orientation of the tip of control-component tool;
   the computer processor is further configured to:
      determine the orientation of the tip of the control-component tool based upon the inertial-measurement-unit data, and
      move the robotic unit in response to the XYZ location and the orientation of the tip of the control-component tool determined by the computer processor; and the robotic unit comprises:
         a tool mount configured to securely hold the one or more tools thereupon;
         at least one multi-jointed arm disposed on a side of the tool mount and configured to moveably support the tool mount;
         a plurality of arm-motors associated with the at least one multi-jointed arm, the plurality of arm-motors being configured to move the tool mount through at least five degrees-of-freedom; and
         one or more mount-motors (36) associated with the tool mount (30) and configured to move the tool (21) with respect to the tool mount (30) through a sixth degree-of-freedom.

In some applications, the control component includes one or more wrist-support elements configured to support a wrist of the user during movement of the control-component arm.

There is further provided, in accordance with some applications of the present invention, apparatus for performing a procedure on a portion of a body of a patient using one or more tools, the apparatus including:
a robotic unit configured to move the tool through six degrees-of-freedom; and
a control component that includes at least one control-component arm configured to be moved by a user, the control-component arm including:
   a control-component tool disposed at the end of the control-component arm, the control-component tool defining a tip;
   three joints;
   three rotary encoders, each of the three rotary encoders coupled to a respective one of the joints and configured to detect movement of the respective joint and to generate rotary-encoder data indicative of an XYZ location of the tip of the control-component tool, in response thereto; and
   an inertial measurement unit including at least one of sensor selected from the group consisting of: a three-axis accelerometer, a three-axis gyroscope, and a three-axis magnetometer,
      the inertial measurement unit being configured to generate inertial-measurement-unit data; and
a computer processor configured to:
   receive the rotary-encoder data and the inertial-measurement-unit data,
   based upon the rotary-encoder data determine the XYZ location of the tip of the control-component tool, and
   move the robotic unit,
wherein:
   the inertial measurement unit is disposed on the control-component tool;
   the inertial-measurement-unit data generated by the inertial measurement unit is indicative of an orientation of the tip of control-component tool;
   the computer processor is further configured to:
      determine the orientation of the tip of the control-component tool based upon the inertial-measurement-unit data, and
      move the robotic unit in response to the XYZ location and the orientation of the tip of the control-component tool determined by the computer processor; and
   the control component includes an in-built stereoscopic display that is configured to display real-time stereoscopic images of the portion of the patient's body and the one or more tools to the user.

In some applications:
the apparatus is for use with at least first and second tools;
the robotic unit includes a first portion configured to move the first tool through six degrees-of-freedom, and a second portion configured to move the second tool through six degrees-of-freedom;
the at least one control-component arm includes two control-component arms, a first one of the control-component arms being configured to be moved by a right hand of the user and a second one of the control-component arms being configured to be moved by a left hand of the user, and
the computer processor is configured to control the first portion of the robotic unit in response to movement of the first one of the control-component arms, and to control the second portion of the robotic unit in response to movement of the second one of the control-component arms.

In some applications, the first and second control-component arms are asymmetric with respect to each other.

In some applications, the apparatus further includes a head-mounted display that is configured to display real-time images of the portion of the patient's body and the one or more tools to the user.

In some applications, the head-mounted display includes a stereoscopic head-mounted display that is configured to display real-time stereoscopic images of the portion of the patient's body and the one or more tools to the user.

There is further provided, in accordance with some applications of the present invention, apparatus for performing a procedure on a portion of a body of a patient using one or more tools, the apparatus including:
a robotic unit configured to move the tool through six degrees-of-freedom; and
a control component that includes at least one control-component arm configured to be moved by a user, the control-component arm including:
   a control-component tool disposed at the end of the control-component arm, the control-component tool defining a tip;
   three joints;
   three rotary encoders, each of the three rotary encoders coupled to a respective one of the joints and configured to detect movement of the respective joint and to generate rotary-encoder data indicative of an XYZ location of the tip of the control-component tool, in response thereto; and
   an inertial measurement unit including at least one of sensor selected from the group consisting of: a three-axis accelerometer, a three-axis gyroscope, and a three-axis magnetometer,
      the inertial measurement unit being configured to generate inertial-measurement-unit data; and
a computer processor configured to:
   receive the rotary-encoder data and the inertial-measurement-unit data,
   based upon the rotary-encoder data determine the XYZ location of the tip of the control-component tool, and
   move the robotic unit,
wherein:
   the inertial measurement unit is disposed on the control-component tool;
   the inertial-measurement-unit data generated by the inertial measurement unit is indicative of an orientation of the tip of control-component tool;
   the computer processor is further configured to:
      determine the orientation of the tip of the control-component tool based upon the inertial-measurement-unit data, and
      move the robotic unit in response to the XYZ location and the orientation of the tip of the control-component tool determined by the computer processor;
   the robotic unit is configured to perform intraocular surgery on an eye of the patient using the one or more tools, and the one or more tools include tips;
   the robotic unit comprises a tool mount configured to securely hold the one or more tools thereupon, and one or more multi-jointed arms disposed on a single side of the tool mount and configured to moveably support the tool mount; and
   the computer processor is configured to drive the robotic unit to perform at least a portion of a procedure on the patient's eye by moving the tip of the tool in a desired manner with respect to the eye such as to perform the portion of the procedure, while entry of the tool into the patient's eye is maintained fixed at the incision point.

The present invention will be more fully understood from the following detailed description of embodiments thereof, taken together with the drawings, in which:

### BRIEF DESCRIPTION OF THE DRAWINGS

Fig. 1A is a schematic illustration of a robotic system that is configured for use in a microsurgical procedure, such as intraocular surgery;
Fig. 1B is a schematic illustration of first and second robotic units of the robotic system that is configured for use in intraocular surgery;
Fig. 2 is a schematic illustration of a robotic unit as shown in Fig. 1B, the robotic unit being shown in the absence of the patient's anatomy;
Figs. 3A, 3B, 3C, and 3D are schematic illustrations of a tool mount of a robotic unit;
Fig. 4 is a schematic illustration of a robotic unit that includes tensioning cables;
Figs. 5A, 5B, and 5C are schematic illustrations showing the range of motion of a tool that is configured to be moved by a robotic unit such as to conform with the normal range of motion through which an ophthalmologist moves surgical tools during manually-performed intraocular surgery;
Figs. 6A and 6B are schematic illustrations of a robotic unit for use in a robotic system;
Fig. 7 is a schematic illustration of a control-component arm configured to be moved by a user and to thereby control movement of an arm of a robotic unit, in accordance with some applications of the present invention;
Fig. 8 is a schematic illustration of a control component;
Figs. 9A and 9B are schematic illustrations of respective views of a control component that includes an in-built stereoscopic vision system; and
Fig. 10 is a schematic illustration of a control component and a head-mounted display for use with the control component.

### DETAILED DESCRIPTION OF EMBODIMENTS

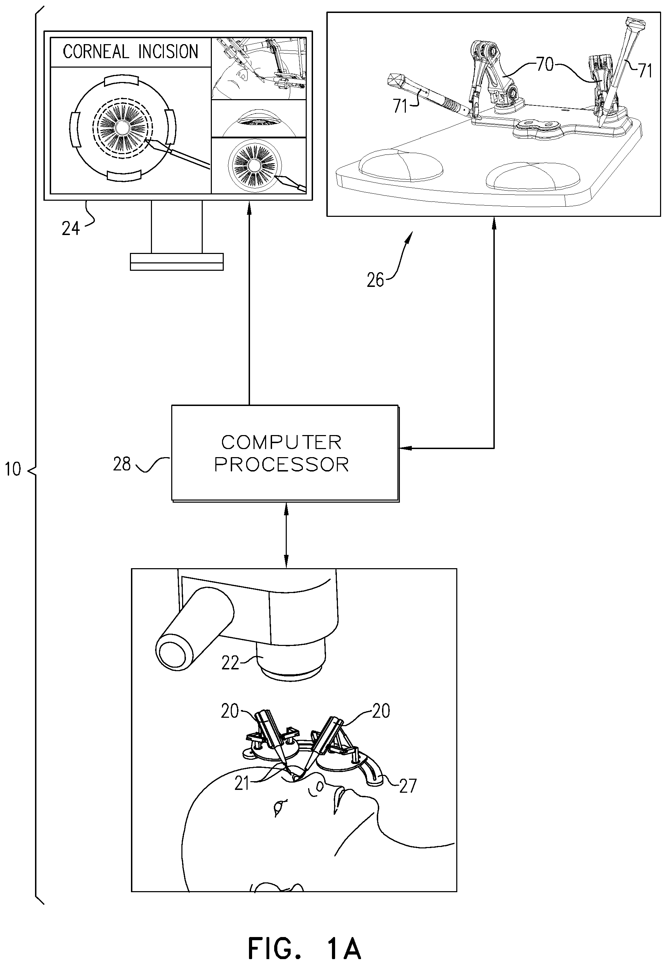

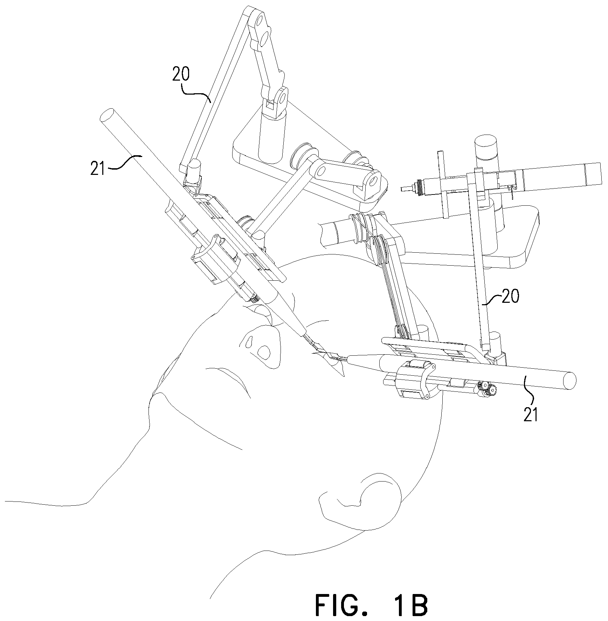

Reference is now made to Fig. 1A, which is a schematic illustration of a robotic system 10 that is configured for use in a microsurgical procedure, such as intraocular surgery. Reference is also made to Fig. 1B, which is a schematic illustration of first and second robotic units 20 of the robotic system. It is noted that the example of robotic units 20 shown in Fig. 1B differs from that shown in Fig. 1A. Typically, robotic system 10 includes first and second robotic units 20 (which are configured to hold tools 21), in addition to an imaging system 22, a display 24 and a control component 26, via which a user (e.g., a healthcare professional) is able to control robotic units 20. Typically, robotic system 10 includes one or more computer processors 28, via which components of the system and a user (e.g., a healthcare professional) operatively interact with each other. For some applications, the first and second robotic units 20 are supported on a support surface 27, as shown. The scope of the present application includes mounting first and second robotic units in any of a variety of different positions with respect to each other.

It is noted that intraocular surgery, and particularly cataract surgery, is characterized by the fact that there is relatively little variability in ocular anatomy and/or geometry from one patient to another. In addition, cataract surgery has a relatively standardized procedural flow, the typical sequence of steps being as noted hereinabove in the Background section. In view of the relative standard dimensions and sequence of steps that are associated with cataract surgery, for some applications of the present invention, robotic system 10 is initially trained to perform one or more steps of cataract surgery in an automated manner, based upon standard ranges of dimensions of respective portions of a human eye. Typically, during the training stage, movement of the robotic units (and/or control of other aspects of the robotic system) is at least partially controlled by a user (e.g., a healthcare professional). For example, the user may receive images of the patient's eye and the robotic units, and/or tools disposed therein, via display 24. Typically, such images are acquired by imaging system 22. For some applications, imaging system 22 is a stereoscopic imaging device and display 24 is a stereoscopic display. Based on the received images, the user typically performs steps of the procedure. For some applications, the user provides commands to the robotic units via control component 26 (which is described in further detail hereinbelow). Typically, such commands include commands that control the position and/or orientation of tools that are disposed within the robotic units, and/or commands that control actions that are performed by the tools. For example, the commands may control a phacoemulsification tool (e.g., the operation mode and/or suction power of the phacoemulsification tool), and/or injector tools (e.g., which fluid (e.g., viscoelastic fluid, saline, etc.) should be injected, and/or at what flow rate). Alternatively or additionally, the user may input commands that control the imaging system (e.g., the zoom, focus, and/or x-y positioning of the imaging system). For some applications , the commands include controlling an IOL-manipulator tool, for example, such that the tool manipulates the IOL inside the eye for precise positioning of the IOL within the eye.

For some applications, computer processor 28 of robotic system 10 analyzes the commands that the user (e.g., the healthcare professional) provides, in order to perform respective steps of the procedure. Typically, based upon the analysis of many such procedures, the computer processor (and/or another computer processor that is associated with the computer processor) determines how to perform one or more steps of cataract surgery in an automated manner, based upon standard ranges of dimensions of respective portions of a human eye. For some applications, the above steps are performed using machine-learning algorithms, such as Linear Regression, Logistic Regression, Decision Tree, Support Vector Machine, Naive Bayes, kNN, K-Means, Random Forest, Dimensionality Reduction Algorithms, and/or Gradient Boosting algorithms. It is noted that typically, the training is not performed using a single robotic system and a single associated computer processor. Rather, many such robotic systems typically provide data that are processed by a centralized computer processor (or a network of computer processors). It is further noted that it is typically the case that, even after an initial training phase, the robotic systems continue to receive further training data as additional procedures are performed on further patients.

For some applications, the computer processor is trained at least partially by one or more human programmers by the human programmers providing the computer processor with programming instructions for how to perform respective steps (and/or portions thereof and/or combinations thereof). For example, one or more human programmers may analyze procedures that are performed on portions of human eyes having standard ranges of dimensions. Based upon the analysis, the one or more human programmers may analyze the motion that a tool undergoes, other functions of the tool, and/or functions of other portions of the robotic system (e.g., functions and/or movements that the imaging system undergoes) as respective steps of the procedure (and/or portions thereof and/or combinations thereof) are performed. The human programmers may then write algorithms that instruct the one or more computer processors to drive the robotic units (and/or other components of the robotic system) to perform the motion and/or functions automatically. For some applications, at least a portions of the algorithms are written by the computer processors analyzing inputs that are input to the computer processor via the control component 26.

For some applications, after the initial training phase, robotic system 10 is used to perform one or more steps of cataract surgery on an eye of a given patient, in an at least partially automated manner. For some such applications, at least one image of the patient's eye is acquired using imaging system 22. For example, the image may be acquired prior to or during one or more steps of cataract surgery being performed on the eye of the patient. Typically, the computer processor analyzes the image in order to determine precise dimensions of the patient's eye, and the steps of the cataract surgery are planned based upon the training and the determined dimensions of the eye. For some applications, the image is further analyzed to determine the position and/or orientation of the eye, and the steps of the cataract surgery are additionally planned based the determined position and/or orientation of the eye. Typically, the planning the steps of the cataract surgery involves planning controlling the positions and orientation of tools via the robotic units, as well as controlling additional aspects of the procedure (such as controlling functions of the tools, and/or controlling functions and/or positioning of the imaging system), as described hereinabove. Subsequent to the planning having been performed, the computer processor typically drives the robotic units and/or additional components of the robotic system to perform the planned steps. For some applications, during the procedure, a user (e.g., a healthcare professional) is able to control movement of the robotic units and/or other aspects of the robotic system. For example, the computer processor may plan a step of the procedure and indicate the planned step to the user (e.g., via display 24). The user may then override and/or refine the planned step of the procedure e.g., via control component 26.

For some applications, any one of the following steps of a cataract procedure (or a portion thereof, and/or a combination thereof) is performed in an automated or semi-automated manner using the techniques described herein: corneal incision (e.g., a bi-planar or tri-planar incision, typically using a keratome blade), viscoelastic injection (including needle insertion via one of the incisions and/or simultaneous injection and retraction of the needle), capsulorhexis, hydrodissection, hydrodelineation, phacoemulsification (including nuclear chopping, and/or ultrasonic emulsification using a phacoemulsification probe), irrigation, aspiration, implantation of an IOL (e.g., using a IOL injection system), viscoelastic removal, wound healing, and/or any combination thereof. For some applications, the performance of the above-described steps includes controlling the position and/or orientation of tools that are disposed within the robotic units, and/or controlling actions that are performed by the tools. For example, controlling a phacoemulsification tool (e.g., the operation mode and/or suction power of the phacoemulsification tool), and/or injector tools (e.g., which fluid (e.g., viscoelastic fluid, saline, etc.) should be injected, and/or at what flow rate). Alternatively or additionally, the performance of the above-described steps may include controlling the imaging system (e.g., the zoom, focus, and/or x-y positioning of the imaging system). For some applications, the performance of the above-described steps includes controlling an IOL-manipulator tool, for example, such that the tool manipulates the IOL inside the eye for precise positioning of the IOL within the eye.

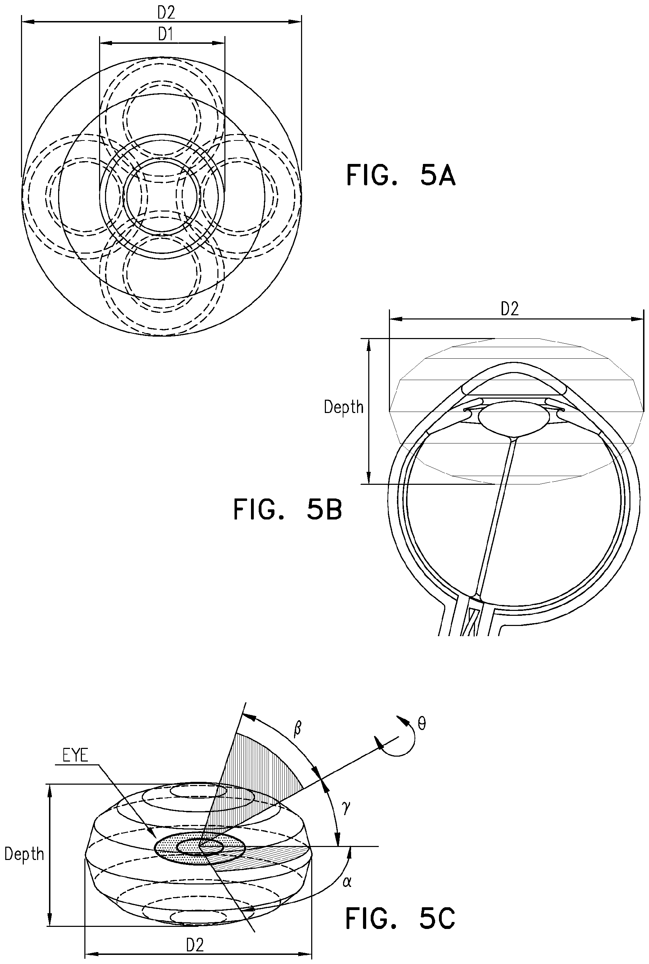

For some applications, the above-described steps of imaging the patient's eye, planning a procedural step, and moving the robotic units and/or controlling other aspects of the robotic system are performed in an iterative manner. Typically, throughout a given procedural step being performed, the patient's eye and the robotic units (and/or tools disposed therein) are continuously imaged in real time. In response to the real-time images, the position and orientation of the tool is automatically updated to correspond with real-time movement of the patient's eye (as described in further detail hereinbelow with reference to Figs. 5A-C). Typically, the position and orientation of the tool is updated by moving the tool via the robotic units. Alternatively or additionally, the computer processor is configured to detect when the eye is at a given position, and to time the performance of certain functions by the robotic units such that they are performed when the eye is at the given position.

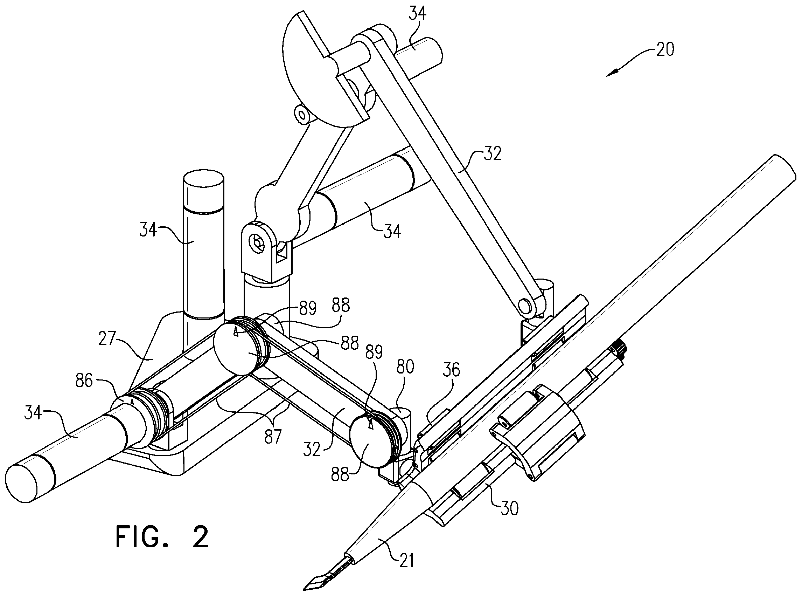

Reference is now made to Fig. 2, which is a schematic illustration of robotic unit 20 as shown in Fig. 1B (the robotic unit being shown in the absence of the patient's anatomy. For some applications, each of the robotic units includes a tool mount 30 configured to securely hold the tool thereupon and to insert the tool into the patient's eye such that entry of the tool into the patient's eye is via an incision point, and the tip of the tool is disposed within the patient's eye. For some applications, two multi-jointed arms 32 are disposed on a single side of the tool mount and are configured to moveably support the tool mount.

Typically, the computer processor detects movement of the patient's eye in three dimensions, by analyzing images acquired by imaging system 22 (which as described hereinabove is typically a stereoscopic imaging system). For some applications, in response to the detected movement of the patient's eye, the computer processor drives the robotic unit to move the tool such that entry of the tool into the patient's eye remains via the incision point even as the patient's eye undergoes the movement in three dimensions. Typically, even as the patient's eye undergoes the movement in three dimensions, the computer processor drives the robotic unit to perform at least a portion of a procedure on the patient's eye by moving the tip of the tool in a desired manner with respect to the eye such as to perform the portion of the procedure, while entry of the tool into the patient's eye is maintained fixed at incision point. In this manner, the robotic unit acts to provide a dynamic remote center of motion that is located at the incision point, and about which motion of the tool is centered. Typically, the remote center of motion moves in coordination with movement of the eye. Alternatively or additionally, the computer processor is configured to detect when the eye is at a given position, and to time the performance of certain functions by the robotic units such that they are performed when the eye is at the given position. For some applications, the above-described steps are performed while the patient's eye is held substantially stationary by virtue of the tool being disposed within the eye. That is to say that, while the eye is held stationary, the computer processor drives the robotic unit to perform at least a portion of a procedure on the patient's eye by moving the tip of the tool in a desired manner with respect to the eye such as to perform the portion of the procedure, while entry of the tool into the patient's eye is maintained fixed at incision point.

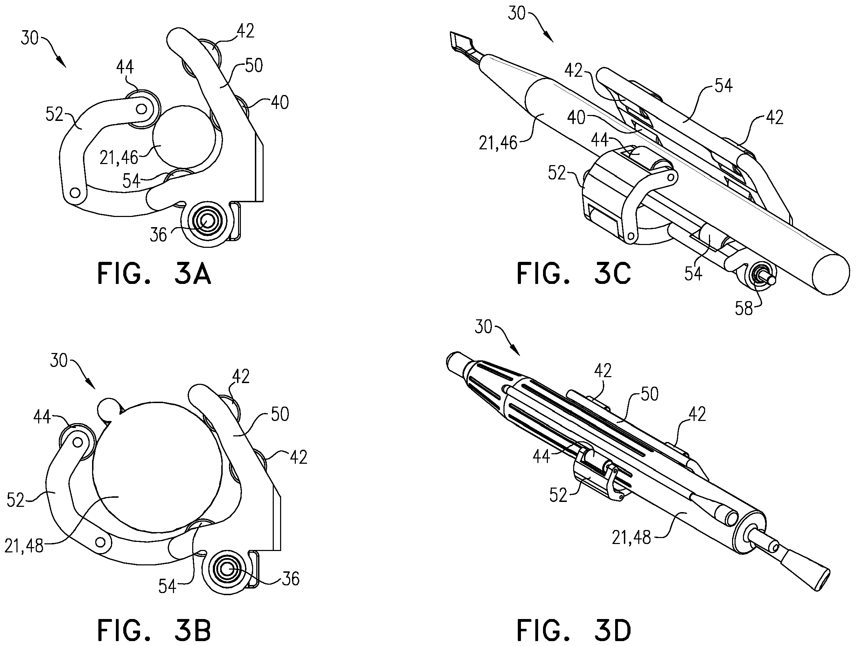

Typically, a plurality of arm-motors 34 are associated with the two multi-jointed arms 32. The plurality of arm-motors move tool mount 30 through five degrees-of-freedom (e.g., movement along the x-, y-, and z-axes, as well as pitch and yaw). For some application, the plurality of arm-motors are configured to move the tool mount (and/or the tool) through more than five degrees-of-freedom. Further typically, one or more mount-motors 36 (shown more clearly in Fig. 3A and 3B) are associated with the tool mount and are configured to move the tool with respect to the tool mount through a sixth degree-of-freedom (e.g., roll). For some applications, the one or more mount-motors associated with the tool mount are configured to move the tool with respect to the tool mount through a further degree-of-freedom.

Reference is now made to Figs. 3A, 3B, 3C, and 3D, which are schematic illustrations of respective views of tool mount 30 of robotic unit 20. Typically, tool mount 30 is configured such that it is configured to house a plurality of differently sized tools. For some applications, the tool mount includes first and second set of rollers 40, 42 that are disposed at fixed positions with respect to each other, and a third roller 44 that is movable with respect to the first and second sets of rollers. Typically, the rollers are mounted on a first support element 50 (which supports the first and second sets of rollers) and a second support element 52 (which supports the third roller), and the second support element is pivotably mounted with respect to the first support element, such that the third roller 44 is movable with respect to the first and second sets of rollers. Thus, as indicated, the tool mount is configured to securely hold tools having different circumferences from each other, by the third roller being moved with respect to the first and second rollers, such that the different combinations of the first, second, and third rollers encompass respective circumferences. For example, a tool having a relatively smaller circumference may be held between the first set of rollers and the third roller (as shown in Figs. 3A and 3C), while a tool having a larger circumference may be held between the second set of rollers and the third roller (as shown in Figs. 3B and 3D). The tool mount is typically configured to securely hold tools having different diameters from each other in such a manner that each of the tools is rollable while disposed inside the tool mount. For some applications, the tool mount is configured to securely hold tools having diameters ranging from 3mm - 20 mm, e.g., 6 mm - 15 mm. For example, Fig. 3A and 3C show the tool mount securely holding a keratome blade 46 (which typically has a diameter of 5-8 mm), while Figs. 3B and 3D show the tool mount securely holding a phacoemulsification probe 48 (which typically has a diameter of 10-14 mm). For some applications, the tool mount is designed in a different manner that allows the tool mount (a) to securely hold tools having different circumferences from each other, while (b) each of the tools is rollable while disposed inside the tool mount.

As noted above, one or more mount-motors 36 are associated with the tool mount and are configured to move the tool with respect to the tool mount through a sixth degree-of-freedom (e.g., roll). For some applications, the mount-motor rotates the tool by rotating a fourth roller 54, via a gear mechanism 58 (gear mechanism 58 being visible in Fig. 3C).

As described above, typically, the rollers are mounted on support elements 50 and 52. For some applications, the support surfaces and the rollers are separable from other portions of the robotic units, and are configured to be sterilized between uses with different patients, and/or are configured to be used for a single procedure before being disposed of. For some applications, the robotic unit defines a seventh degree of freedom, which is typically associated with the tool mount, and which controls movement of a syringe piston (i.e., a syringe plunger), such as to control the injection of fluid, saline, viscoelastic, etc. Alternatively or additionally, the robotic unit includes addition degrees-of-freedom associated with additional functions of tools 21.

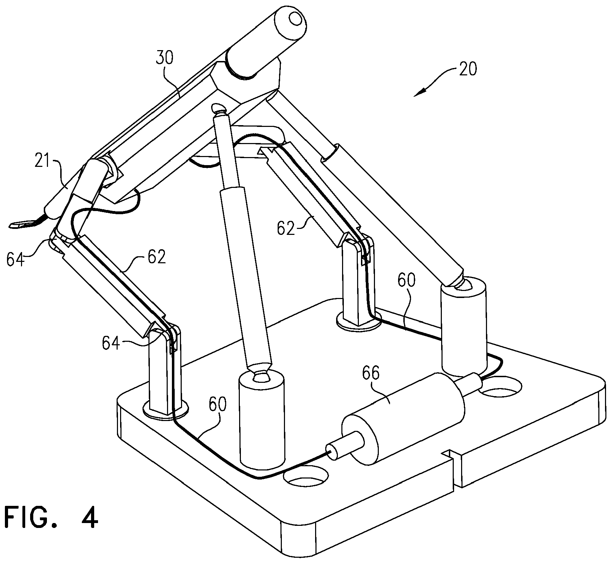

Reference is now made to Fig. 4, which is a schematic illustration of a robotic unit 20 that includes one or more tensioning cables 60. It is noted that the example of robotic unit 20 shown in Fig. 4 is generally similar to that shown in Fig. 1A, and is different from that shown in Figs. 1B and 2. In the example shown in Fig. 4, there are respective multi-jointed arms 62 disposed on each side of tool mount 30. In general, features of robotic unit 20 as shown in Fig. 4 and techniques that are described for use therewith are applicable to other examples of robotic unit 20, and vice versa.

It is typically the case that movements of the robotic unit are controlled via motors and gear mechanisms. In general, such gear mechanisms have some freedom of movement even if there is no input to the gear mechanism, in order to prevent the gear mechanisms from getting stuck. Typically, this can result in backlash of the gear mechanism after movement of the gear mechanism, which can result in imprecise positioning of the tool disposed within the robotic unit with respect to the patient's eye. Therefore, for some applications, one or more tensioning cables are placed around several joints 64 of the multi-jointed arms, such as to create tension on the joints. For some applications, a tensioning-cable motor 66 is configured to apply tension to the tensioning cable. Typically, the tensioning-cable motor 66 applies tension at a constant current and/or torque (e.g., by using a constant torque motor as tensioning-cable motor 66), such as to generate a constant tension on the tensioning cable. Typically, this reduces (or eliminates) backlash in the multi-jointed arms, relative to if the tensioning cable were not to be used. Alternatively or additionally, other techniques are used for reducing (or eliminating) backlash in the multi-jointed arms. For example, an anti-backlash gear mechanism may be used.

Reference is now made to Figs. 5A, 5B, and 5C, which are schematic illustrations showing the range of motion of a tool that is configured to be moved by robotic unit 20 such as to conform with the normal range of motion through which an ophthalmologist moves surgical tools during manually-performed intraocular surgery. Referring to Fig. 5A, the diameter D1 of the working space within which cataract surgery is typically performed is typically between 10 and 15 mm, e.g., approximately 12 mm. Typically, over the course of a procedure this working space will move within a circle having a diameter D2 of between 20 mm and 30 mm, e.g. approximately 25 mm. This is due to movement of the eye within the circle, as indicated by the dashed circles indicating the range of eye motion in each direction. Referring to Fig. 5B, the depth of the working space within which cataract surgery is typically performed is typically between 10 mm and 20 mm, e.g., approximately 15 mm.

For some applications, robotic system 10 includes first and second robotic units 20, such as to replicate the standard surgical practice in which an ophthalmologist performs the procedure holding respective tools in her/his left and right hands, each of the tools being inserted into the eye from a respective angle. The use of first and second robotic unit in this manner is illustrated in Figs. 1A and 1B. As described above, typically, the tool mount of the robotic unit is movable through at least five degrees-of-freedom. For some applications, the robotic unit is configured to move the tool mount through five degrees-of-freedom, such that the tool moves along the x-, y-, and z-axes, as well as through pitch and yaw angular movements. Referring to Fig. 5C, typically, the robotic unit is configured to move the tool mount through a yaw angular rotation *alpha* of plus/minus 25 degrees from a central orientation. For some applications, the robotic unit is configured to move the tool mount through a pitch angular rotation *beta* of 60 degrees. Typically movement of the tool mount through the pitch angular rotation *beta* is performed starting from a starting pitch *gamma* of approximately 20 degrees (e.g., between 15 and 25 degrees) from the x-y plane (as indicated in Fig. 5C). Further typically, the robotic unit is configured to move the tool with respect to the tool mount through a sixth degree-of-freedom (e.g., roll). For some applications, the robotic unit is configured to roll the tool with respect to the tool mount, such that the tool has a range of roll angular rotation *theta* of plus/minus 80 degrees from a central position, as indicated in Fig. 5C.

Typically, throughout a given procedural step being performed, the patient's eye and the robotic units (and/or tools disposed therein) are continuously imaged in real time. In response to the real-time images, the position and orientation of the tool is updated to correspond with real-time movement of the patient's eye (as described in further detail hereinbelow). Typically, the position and orientation of the tool is updated by moving the tool via the robotic units. For some applications, during a procedure (or a step thereof), the computer processor detects movement of the patient's eye in three dimensions, by analyzing images acquired by imaging system 22 (which as described hereinabove is typically a stereoscopic imaging system). For some such applications, in response to the detected movement of the patient's eye, the computer processor drives the robotic unit to move the tool such that entry of the tool into the patient's eye remains via the incision point even as the patient's eye undergoes the movement in three dimensions. Typically, even as the patient's eye undergoes the movement in three dimensions, the computer processor drives the robotic unit to perform at least a portion of a procedure on the patient's eye by moving the tip of the tool in a desired manner with respect to the eye such as to perform the portion of the procedure, while entry of the tool into the patient's eye is maintained fixed at incision point. Alternatively or additionally, the computer processor is configured to detect when the eye is at a given position, and to time the performance of certain functions by the robotic units such that they are performed when the eye is at the given position.

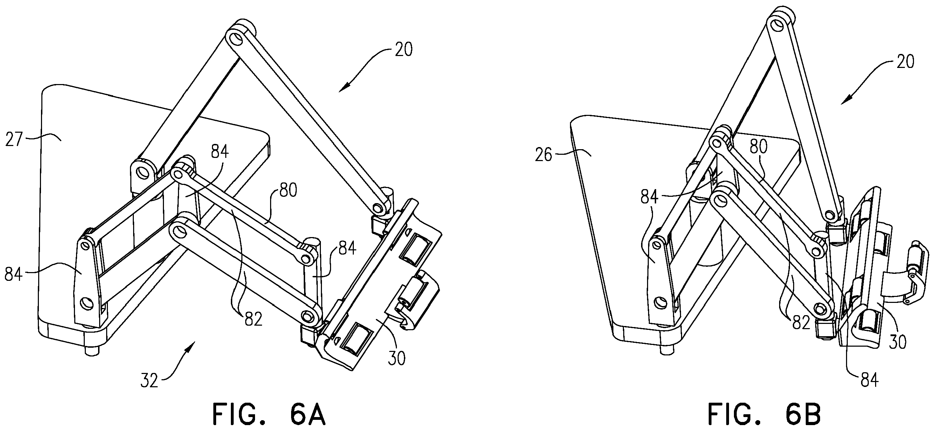

Reference is now made to Figs. 6A and 6B, which are schematic illustrations of an alternative example of a robotic unit 20 for use in robotic system 10. It may be observed that in Figs. 6A-B, each bar 80 of at least one of the multi-jointed arms 32 of the robotic system includes two parallel beams 82 that extend between vertical links 84 that are disposed at each joint. For some applications, the arrangement of parallel beams and vertical links results in the ends of each of the links of a given multi-jointed arm remaining parallel with each other, even as the arm moves (as shown in the transition from Fig. 6A to Fig. 6B). In turn, this prevents the tool mount from being rolled with respect to support surface 27. For some applications, each of the multi-jointed arms is configured in the above-described manner. Alternatively, only one of the multi-jointed arms is configured in the above-described manner.

Reference is again made to Fig. 2, which shows robotic unit 20. For some applications, as an alternative to the arrangement shown in Figs. 6A-B, one or more of the multi-jointed arms is stabilized using a pulley and cable system as shown in Fig. 2. Typically, the multi-jointed arm includes a pulley 86 that is disposed at an end of the arm that is closest to support surface 27. Pulley 86 is held in a fixed and non-rotatable position with respect to support surface 27. Additional pulleys 88 are disposed at each additional link 84 (shown in Fig. 6) of the multi-jointed arm. A loop of cable 87 having a fixed length is typically disposed between each adjacent pair of pulleys (including pulley 86 and additional pulleys 88). The additional pulleys are free to rotate around their axis. However, since the lengths of the loops of cables are fixed and pulley 86 is held in a fixed and non-rotatable position with respect to support surface 27 this results in additional pulleys 88 facing in a fixed direction (indicated by arrow 89) even as the multi-jointed arm moves. For some applications, the above-described arrangement results in the tool mount being prevented from rolling with respect to support surface 27. For some applications, each of the multi-jointed arms is configured in the above-described manner. Alternatively, only one of the multi-jointed arms is configured in the above-described manner.

It is noted that, using a parallel beam system to prevent rolling of the tool mount with respect to support surface 27 (as shown in Fig. 6A-B) typically limits the range of motion of the multi-jointed arms, due to parallel beams colliding with each other. For example, the multi-jointed arm typically cannot be folded much more than the configuration shown in Fig. 6B. By contrast, the arm is not limited in this manner when the arm includes a pulley and cable system as shown in Fig. 2. Typically, when configured as shown in Fig. 2, adjacent portions of the arm can be folded to be almost parallel with each other (e.g., such that the adjacent portions of the arm are disposed at an angle of less than 10 degrees with respect to each other).

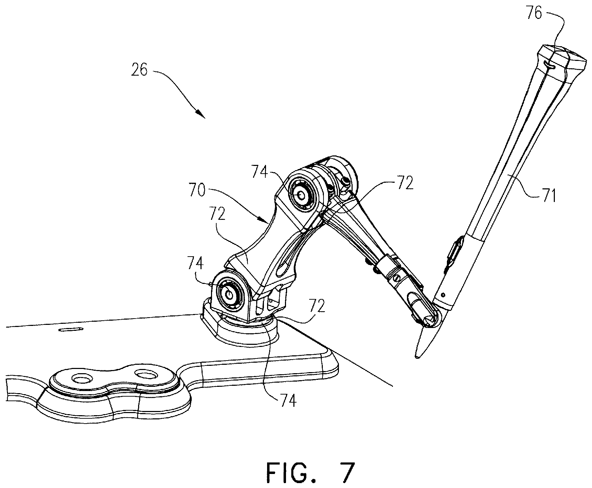

Reference is now made to Fig. 7, which is a schematic illustration of control-component arm 70 of a control component 26, which is configured to be moved by a user and to thereby control movement of an arm of a robotic unit 20, in accordance with some applications of the present invention. In accordance with the above description of Figs. 1A-1B, for some applications, control component 26 is used to control the robotic units during a training phase, when the robotic system is being trained to perform a procedure (and/or steps thereof). Alternatively or additionally, the control component 26 is used to control the robotic units when a procedure is subsequently performed upon a given patient, for example, in order to override and/or to refine a step of the procedure that the robotic system is going to perform. Further alternatively or additionally, by default, the control component is used to control movement of an arm of the robotic unit when a procedure is performed upon a given patient.

Typically, the control component includes first and second control-component arms 70 that are configured to correspond to respective robotic units 20 of the robotic system, as shown in Fig. 1A. The control-component arms are configured to hold respective control-component tools 71 therein (in order to replicate the arms of the robotic units), also as shown in Fig. 1A. (In Fig. 7, only one control-component arm is shown and the control-component arm 70 is shown with a control-component tool 71.)

According to the invention, each of control-component arms 70 includes three joints 72, and a respective rotary encoder 74 coupled to each one of the three joints. The rotary encoders are configured to detect movement of the respective joints and to generate rotary-encoder data in response thereto. The control-component arm additionally includes an inertial-measurement unit 76 that includes a three-axis accelerometer, a three-axis gyroscope, and/or a three-axis magnetometer. The inertial-measurement unit generates inertial-measurement-unit data relating to a three-dimensional orientation of the control-component arm, in response to the control-component arm being moved. Computer processor 28 receives the rotary-encoder data and the inertial-measurement-unit data. The computer processor determines the XYZ location of the tip of the control-component tool 71, based upon the rotary-encoder data, and determines the orientation of the tip of control-component tool 71 (e.g., the 3 Euler angles of orientation, and/or another representation of orientation) based upon the inertial-measurement-unit data. Thus, based upon the combination of the rotary-encoder data and the inertial-measurement-unit data, the computer processor is configured to determine the XYZ location and orientation of the tip of the control-component tool.

The computer processor drives the robotic unit such that the tip of the actual tool that is being used to perform the procedure tracks the movements of the tip of the control-component tool. Typically, incorporating an inertial-measurement unit to detect the three-dimensional orientation of the control-component arm allows the user to control movement of the robotic unit using a reduced number of sensors, relative to if rotary encoders were used to detect motion of the control-component arm in all six degrees-of-freedom. Further typically, reducing the number of rotary encoders that are used tends to reduce the overall complexity of the control component, since introducing additional rotary encoders would require additional wires to pass through rotating joints.

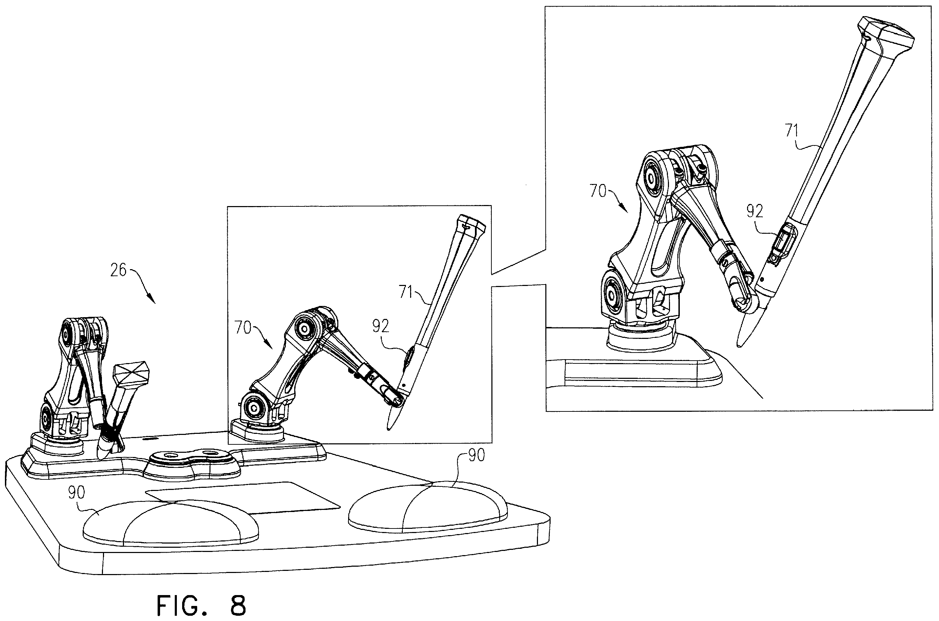

Reference is now made to Fig. 8, which is a schematic illustration of control component 26. For some applications, the control component includes one or more ergonomic features. For example, the control component may include wrist-support elements 90, such as cushions or gel-filled pads. For some applications, the left and right control-component arms 70 are designed asymmetrically, such that the user is able to grip and move the arms with their left and right hands in a comfortable manner. As described hereinabove, typically, the control-component arms are used to control movement of an arm of a robotic unit 20. For example, as described, based upon the combination of the rotary-encoder data and the inertial-measurement-unit data, the computer processor may be configured to determine the XYZ location and orientation of the tip of the control-component tool. For some applications, the control component arms include additional controls. For example, the control-component arm may include a button 92 that is configured to activate a tool, such as forceps.

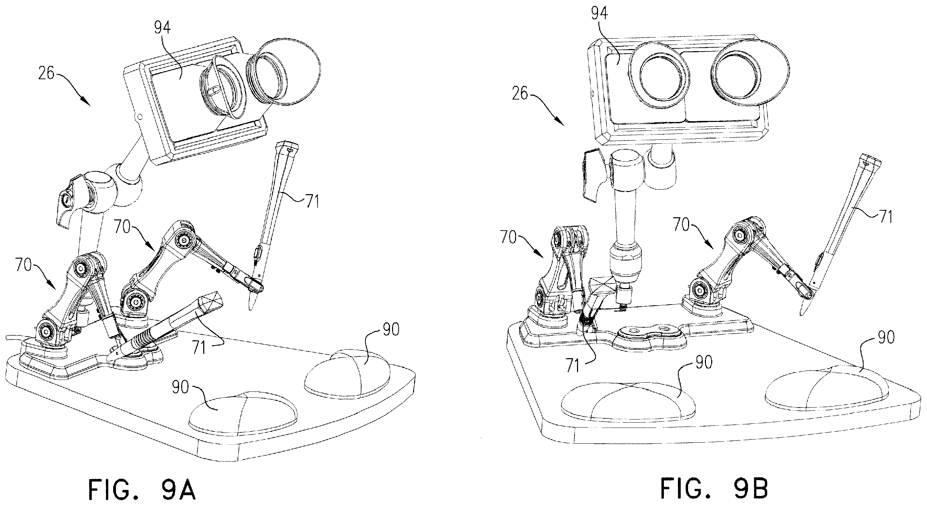

Reference is now made to Figs. 9A and 9B, which are schematic illustrations of respective views of control component 26, the control component including an in-built stereoscopic vision system 94. As described hereinabove, for some applications, imaging system 22 is a stereoscopic imaging device. For some such applications, based upon images acquired by the imaging system, real-time stereoscopic images are displayed to the user on stereoscopic system 94, which is in-built to the control component. Based on the received images, the user typically performs steps of the procedure, typically by providing commands to the robotic units via control-arms 70 of control component 26.

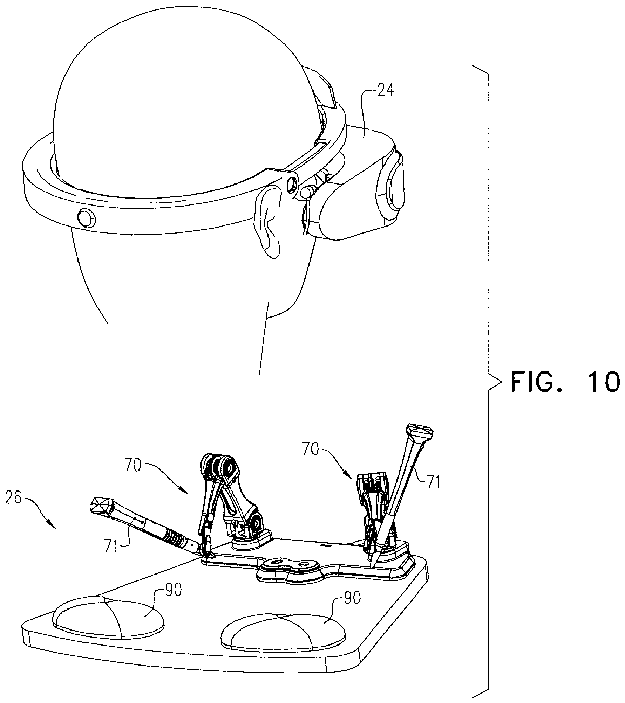

Reference is now made to Fig. 10, which is a schematic illustration of control component 26, and a head-mounted display 96 for use with the control component. As described hereinabove, for some applications, based upon images acquired by imaging system 22, real-time images are displayed to the user. For some applications, the images are displayed on head-mounted display 96. Typically, imaging system acquires stereoscopic images, and the head-mounted display displays stereoscopic images to the user. Based on the received images, the user typically performs steps of the procedure, typically by providing commands to the robotic units via control-arms 70 of control component 26.

Although some applications of the present invention are described with reference to cataract surgery, the scope of the present application includes applying the apparatus described herein to other medical procedures, *mutatis mutandis.* In particular, the apparatus and methods described herein to other medical procedures may be applied to other microsurgical procedures, such as general surgery, orthopedic surgery, gynecological surgery, otolaryngology, neurosurgery, oral and maxillofacial surgery, plastic surgery, podiatric surgery, vascular surgery, and/or pediatric surgery that is performed using microsurgical techniques. For some such applications, the imaging system includes one or more microscopic imaging units.

It is noted that the scope of the present application includes applying the apparatus described herein to intraocular procedures, other than cataract surgery, *mutatis mutandis.* Such procedures may include collagen crosslinking, endothelial keratoplasty (e.g., DSEK, DMEK, and/or PDEK), DSO (descemet stripping without transplantation), laser assisted keratoplasty, keratoplasty, LASIK/PRK, SMILE, pterygium, ocular surface cancer treatment, secondary IOL placement (sutured, transconjunctival, etc.), iris repair, IOL reposition, IOL exchange, superficial keratectomy, Minimally Invasive Glaucoma Surgery (MIGS), limbal stem cell transplantation, astigmatic keratotomy, Limbal Relaxing Incisions (LRI), amniotic membrane transplantation (AMT), glaucoma surgery (e.g., trabs, tubes, minimally invasive glaucoma surgery), automated lamellar keratoplasty (ALK), anterior vitrectomy, and/or pars plana anterior vitrectomy.

Applications of the invention described herein can take the form of a computer program product accessible from a computer-usable or computer-readable medium (e.g., a non-transitory computer-readable medium) providing program code for use by or in connection with a computer or any instruction execution system, such as computer processor 28. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W), DVD, and a USB drive.

A data processing system suitable for storing and/or executing program code will include at least one processor (e.g., computer processor 28) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object-oriented programming language such as Java, Smalltalk, C++ or the like and conventional procedural programming languages, such as the C programming language or similar programming languages.

It will be understood that the algorithms described herein, can be implemented by computer program instructions. These computer program instructions may be provided to a processor of a general-purpose computer, special purpose computer, or other programmable data processing apparatus to produce a machine, such that the instructions, which execute via the processor of the computer (e.g., computer processor 28) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

Computer processor 28 is typically a hardware device programmed with computer program instructions to produce a special purpose computer. For example, when programmed to perform the algorithms described with reference to the Figures, computer processor 28 typically acts as a special purpose robotic-system computer processor. Typically, the operations described herein that are performed by computer processor 28 transform the physical state of a memory, which is a real physical article, to have a different magnetic polarity, electrical charge, or the like depending on the technology of the memory that is used. For some applications, operations that are described as being performed by a computer processor are performed by a plurality of computer processors in combination with each other.

It will be appreciated by persons skilled in the art that the present invention is not limited to what has been particularly shown and described hereinabove. Rather, the scope of the present invention is defined by the appended claims, which may include both combinations and subcombinations of the various features described hereinabove, as well as variations and modifications thereof that are not in the prior art, which would occur to persons skilled in the art upon reading the foregoing description.

## Claims

1. Apparatus for performing a procedure on a portion of a body of a patient using one or more tools (21), the apparatus comprising:
a robotic unit (20) configured to move the tool through six degrees-of-freedom; and
a control component (26) that comprises at least one control-component arm (70) configured to be moved by a user, the control-component arm (70) comprising:
a control-component tool (71) disposed at the end of the control-component arm (70), the control-component tool (71) defining a tip;
three joints;
three rotary encoders (74), each of the three rotary encoders coupled to a respective one of the joints and configured to detect movement of the respective joint and to generate rotary-encoder data indicative of an XYZ location of the tip of the control-component tool (71), in response thereto; and
an inertial measurement unit (76) comprising at least one of sensor selected from the group consisting of: a three-axis accelerometer, a three-axis gyroscope, and a three-axis magnetometer,
the inertial measurement unit (76) being configured to generate inertial-measurement-unit data; and
a computer processor (28) configured to:
receive the rotary-encoder data and the inertial-measurement-unit data,
based upon the rotary-encoder data determine the XYZ location of the tip of the control-component tool (71), and
move the robotic unit (20),
**characterized in that**:
the inertial measurement unit (76) is disposed on the control-component tool (71);
the inertial-measurement-unit data generated by the inertial measurement unit (76) is indicative of an orientation of the tip of control-component tool (71);
the computer processor (28) is further configured to:
determine the orientation of the tip of the control-component tool (71) based upon the inertial-measurement-unit data, and
move the robotic unit (20) in response to the XYZ location and the orientation of the tip of the control-component tool (71) determined by the computer processor (28); and
the robotic unit comprises:
a tool mount (30) configured to securely hold the one or more tools (21) thereupon;
at least one multi-jointed arm (32) disposed on a side of the tool mount (30) and configured to moveably support the tool mount (30);
a plurality of arm-motors (34) associated with the at least one multi-jointed arm (32), the plurality of arm-motors (34) being configured to move the tool mount (30) through at least five degrees-of-freedom; and
one or more mount-motors (36) associated with the tool mount (30) and configured to move the tool (21) with respect to the tool mount (30) through a sixth degree-of-freedom.

2. The apparatus according to claim 1, wherein the control component (26) comprises one or more wrist-support elements (90) configured to support a wrist of the user during movement of the control-component arm (70).

3. Apparatus for performing a procedure on a portion of a body of a patient using one or more tools (21), the apparatus comprising:
a robotic unit (20) configured to move the tool through six degrees-of-freedom; and
a control component (26) that comprises at least one control-component arm (70) configured to be moved by a user, the control-component arm (70) comprising:
a control-component tool (71) disposed at the end of the control-component arm (70), the control-component tool (71) defining a tip;
three joints;
three rotary encoders (74), each of the three rotary encoders coupled to a respective one of the joints and configured to detect movement of the respective joint and to generate rotary-encoder data indicative of an XYZ location of the tip of the control-component tool (71), in response thereto; and
an inertial measurement unit (76) comprising at least one of sensor selected from the group consisting of: a three-axis accelerometer, a three-axis gyroscope, and a three-axis magnetometer,
the inertial measurement unit (76) being configured to generate inertial-measurement-unit data; and
a computer processor (28) configured to:
receive the rotary-encoder data and the inertial-measurement-unit data,
based upon the rotary-encoder data determine the XYZ location of the tip of the control-component tool (71), and
move the robotic unit (20),
**characterized in that**:
the inertial measurement unit (76) is disposed on the control-component tool (71);
the inertial-measurement-unit data generated by the inertial measurement unit (76) is indicative of an orientation of the tip of control-component tool (71);
the computer processor (28) is further configured to:
determine the orientation of the tip of the control-component tool (71) based upon the inertial-measurement-unit data, and
move the robotic unit (20) in response to the XYZ location and the orientation of the tip of the control-component tool (71) determined by the computer processor (28); and
the control component comprises an in-built stereoscopic display (94) that is configured to display real-time stereoscopic images of the portion of the patient's body and the one or more tools (21) to the user.

4. The apparatus according to any one of claims 1-3, wherein:
the apparatus is for use with at least first and second tools (21);
the robotic unit (20) comprises a first portion configured to move the first tool through six degrees-of-freedom, and a second portion configured to move the second tool through six degrees-of-freedom;
the at least one control-component arm (70) comprises two control-component arms (70), a first one of the control-component arms (70) being configured to be moved by a right hand of the user and a second one of the control-component arms (70) being configured to be moved by a left hand of the user, and
wherein the computer processor (28) is configured to control the first portion of the robotic unit (20) in response to movement of the first one of the control-component arms (70), and to control the second portion of the robotic unit (20) in response to movement of the second one of the control-component arms (70).

5. The apparatus according to claim 4, wherein the first and second control-component arms (70) are asymmetric with respect to each other.

6. The apparatus according to any one of claims 1-3, further comprising a head-mounted display (96) that is configured to display real-time images of the portion of the patient's body and the one or more tools (21) to the user.

7. The apparatus according to claim 6, wherein the head-mounted display (96) comprises a stereoscopic head-mounted display that is configured to display real-time stereoscopic images of the portion of the patient's body and the one or more tools (21) to the user.

8. The apparatus according to claim 1, wherein the plurality of arm-motors (34) are configured to move the tool mount (30) along x-, y-, and z-axes, as well as through pitch and yaw angular rotations.

9. The apparatus according to claim 1, wherein the one or more tools (21) comprise two or more tools, each of the two or more tools having respective circumferences that differ from each other, and wherein the tool mount (30) is configured to securely hold each of the two or more tools thereupon.

10. The apparatus according to claim 1, wherein the one or more mount-motors (36) associated with the tool mount (30) are configured to move the tool (21) with respect to the tool mount (30) through a further degree-of-freedom.

11. The apparatus according to claim 1, wherein the tool mount (30) is configured to securely hold the one or more tools (21) thereupon, while allowing the one or more tools (21) to be rolled with respect to the tool mount (30).

12. Apparatus for performing a procedure on a portion of a body of a patient using one or more tools (21), the apparatus comprising:
a robotic unit (20) configured to move the tool through six degrees-of-freedom; and
a control component (26) that comprises at least one control-component arm (70) configured to be moved by a user, the control-component arm (70) comprising:
a control-component tool (71) disposed at the end of the control-component arm (70), the control-component tool (71) defining a tip;
three joints;
three rotary encoders (74), each of the three rotary encoders coupled to a respective one of the joints and configured to detect movement of the respective joint and to generate rotary-encoder data indicative of an XYZ location of the tip of the control-component tool (71), in response thereto; and
an inertial measurement unit (76) comprising at least one of sensor selected from the group consisting of: a three-axis accelerometer, a three-axis gyroscope, and a three-axis magnetometer,
the inertial measurement unit (76) being configured to generate inertial-measurement-unit data; and
a computer processor (28) configured to:
receive the rotary-encoder data and the inertial-measurement-unit data,
based upon the rotary-encoder data determine the XYZ location of the tip of the control-component tool (71), and
move the robotic unit (20),
**characterized in that**:
the inertial measurement unit (76) is disposed on the control-component tool (71);
the inertial-measurement-unit data generated by the inertial measurement unit (76) is indicative of an orientation of the tip of control-component tool (71);
the computer processor (28) is further configured to:
determine the orientation of the tip of the control-component tool (71) based upon the inertial-measurement-unit data, and
move the robotic unit (20) in response to the XYZ location and the orientation of the tip of the control-component tool (71) determined by the computer processor (28);
the robotic unit (20) is configured to perform intraocular surgery on an eye of the patient using the one or more tools (21), and the one or more tools (21) include tips;
the robotic unit (20) comprises a tool mount (30) configured to securely hold the one or more tools (21) thereupon, and one or more multi-jointed arms (32) disposed on a single side of the tool mount (30) and configured to moveably support the tool mount (30); and
the computer processor (28) is configured to drive the robotic unit to perform at least a portion of a procedure on the patient's eye by moving the tip of the tool (21) in a desired manner with respect to the eye such as to perform the portion of the procedure, while entry of the tool (21) into the patient's eye is maintained fixed at the incision point.

13. The apparatus according to claim 12, wherein the one or more multi-jointed arms (32) are configured to move the tool mount (30) along x-, y-, and z-axes, as well as through pitch and yaw angular rotations, and wherein the computer processor (28) is configured to drive the one or more multi-jointed arms (32) to move the tool mount along x-, y-, and z-axes, as well as through pitch and yaw angular rotations, while entry of the one or more tools (21) into the patient's eye is maintained fixed at the incision point.

14. The apparatus according to claim 12, wherein the one or more multi-jointed arms (32) are configured to move the tool mount (30) along x-, y-, and z-axes, as well as through pitch and yaw angular rotations, and wherein the computer processor (28) is configured to drive the tool (21) to roll with respect to the tool mount (30), while entry of the tool (21) into the patient's eye is maintained fixed at the incision point.

## Patentansprüche

1. Vorrichtung zum Durchführen eines Eingriffs an einem Teil eines Körpers eines Patienten unter Verwendung eines oder mehrerer Werkzeuge (21), wobei die Vorrichtung umfasst:
eine Robotereinheit (20), die dazu konfiguriert ist, das Werkzeug über sechs Freiheitsgrade zu bewegen; und
eine Steuerkomponente (26), die mindestens einen Steuerkomponentenarm (70) umfasst, der dazu konfiguriert ist, von einem Benutzer bewegt zu werden, wobei der Steuerkomponentenarm (70) umfasst:
ein Steuerkomponentenwerkzeug (71), das am Ende des Steuerkomponentenarms (70) angeordnet ist, wobei das Steuerkomponentenwerkzeug (71) eine Spitze definiert;
drei Gelenke;
drei Drehgeber (74), wobei jeder der drei Drehgeber mit einem jeweiligen der Gelenke gekoppelt ist und dazu konfiguriert ist, die Bewegung des jeweiligen Gelenks zu erkennen und Drehgeberdaten, die für eine XYZ-Position der Spitze des Steuerkomponentenwerkzeugs (71) bezeichnend sind, als Reaktion darauf zu generieren; und
eine Trägheitsmesseinheit (76), die mindestens einen von einem Sensor umfasst, der ausgewählt ist aus der Gruppe bestehend aus: einem dreiachsigen Beschleunigungsmesser, einem dreiachsigen Gyroskop und einem dreiachsigen Magnetometer,
wobei die Trägheitsmesseinheit (76) dazu konfiguriert ist, Trägheitsmesseinheitsdaten zu generieren; und
einen Computerprozessor (28), der konfiguriert ist zum:
Empfangen der Drehgeberdaten und der Trägheitsmesseinheitsdaten,
Bestimmen, auf der Grundlage der Drehgeberdaten, der XYZ-Position der Spitze des Steuerkomponentenwerkzeugs (71) und
Bewegen der Robotereinheit (20), **dadurch gekennzeichnet, dass**:
die Trägheitsmesseinheit (76) am Steuerkomponentenwerkzeug (71) angeordnet ist;
die von der Trägheitsmesseinheit (76) generierten Trägheitsmesseinheitsdaten für eine Ausrichtung der Spitze des Steuerkomponentenwerkzeugs (71) bezeichnend sind;
der Computerprozessor (28) ferner konfiguriert ist zum:
Bestimmen der Ausrichtung der Spitze des Steuerkomponentenwerkzeugs (71) auf der Grundlage der Trägheitsmesseinheitsdaten und Bewegen der Robotereinheit (20) als Reaktion auf die XYZ-Position und die Ausrichtung der Spitze des Steuerkomponentenwerkzeugs (71), die vom Computerprozessor (28) bestimmt werden; und
die Robotereinheit umfasst:
eine Werkzeughalterung (30), die dazu konfiguriert ist, das eine oder die mehreren Werkzeuge (21) sicher darauf zu halten;
mindestens einen mehrgelenkigen Arm (32), der an einer Seite der Werkzeughalterung (30) angeordnet ist und dazu konfiguriert ist, die Werkzeughalterung (30) beweglich zu stützen;
eine Vielzahl von Armmotoren (34), die dem mindestens einen mehrgelenkigen Arm (32) zugeordnet sind, wobei die Vielzahl von Armmotoren (34) dazu konfiguriert ist, die Werkzeughalterung (30) über mindestens fünf Freiheitsgrade zu bewegen; und
einen oder mehrere Halterungsmotoren (36), die der Werkzeughalterung (30) zugeordnet sind und dazu konfiguriert sind, das Werkzeug (21) in Bezug auf die Werkzeughalterung (30) über einen sechsten Freiheitsgrad zu bewegen.

2. Vorrichtung nach Anspruch 1, wobei die Steuerkomponente (26) ein oder mehrere Handgelenkstützelemente (90) umfasst, die dazu konfiguriert sind, ein Handgelenk des Benutzers während der Bewegung des Steuerkomponentenarms (70) zu stützen.

3. Vorrichtung zum Durchführen eines Eingriffs an einem Teil eines Körpers eines Patienten unter Verwendung eines oder mehrerer Werkzeuge (21), wobei die Vorrichtung umfasst:
eine Robotereinheit (20), die dazu konfiguriert ist, das Werkzeug über sechs Freiheitsgrade zu bewegen; und
eine Steuerkomponente (26), die mindestens einen Steuerkomponentenarm (70) umfasst, der dazu konfiguriert ist, von einem Benutzer bewegt zu werden, wobei der Steuerkomponentenarm (70) umfasst:
ein Steuerkomponentenwerkzeug (71), das am Ende des Steuerkomponentenarms (70) angeordnet ist, wobei das Steuerkomponentenwerkzeug (71) eine Spitze definiert;
drei Gelenke;
drei Drehgeber (74), wobei jeder der drei Drehgeber mit einem jeweiligen der Gelenke gekoppelt ist und dazu konfiguriert ist, die Bewegung des jeweiligen Gelenks zu erkennen und Drehgeberdaten, die für eine XYZ-Position der Spitze des Steuerkomponentenwerkzeugs (71) bezeichnend sind, als Reaktion darauf zu generieren; und
eine Trägheitsmesseinheit (76), die mindestens einen von einem Sensor umfasst, der ausgewählt ist aus der Gruppe bestehend aus: einem dreiachsigen Beschleunigungsmesser, einem dreiachsigen Gyroskop und einem dreiachsigen Magnetometer,
wobei die Trägheitsmesseinheit (76) dazu konfiguriert ist, Trägheitsmesseinheitsdaten zu generieren; und
einen Computerprozessor (28), der konfiguriert ist zum:
Empfangen der Drehgeberdaten und der Trägheitsmesseinheitsdaten,
Bestimmen, auf der Grundlage der Drehgeberdaten, der XYZ-Position der Spitze des Steuerkomponentenwerkzeugs (71) und
Bewegen der Robotereinheit (20),
**dadurch gekennzeichnet, dass**:
die Trägheitsmesseinheit (76) am Steuerkomponentenwerkzeug (71) angeordnet ist;
die von der Trägheitsmesseinheit (76) generierten Trägheitsmesseinheitsdaten für eine Ausrichtung der Spitze des Steuerkomponentenwerkzeugs (71) bezeichnend sind;
der Computerprozessor (28) ferner konfiguriert ist zum:
Bestimmen der Ausrichtung der Spitze des Steuerkomponentenwerkzeugs (71) auf der Grundlage der Trägheitsmesseinheitsdaten, und
Bewegen der Robotereinheit (20) als Reaktion auf die XYZ-Position und die Ausrichtung der Spitze des Steuerkomponentenwerkzeugs (71), die vom Computerprozessor (28) bestimmt werden; und
die Steuerkomponente eine eingebaute stereoskopische Anzeige (94) umfasst, die dazu konfiguriert ist, dem Benutzer stereoskopische Echtzeitbilder des Teils des Körpers des Patienten und des einen oder der mehreren Werkzeuge (21) anzuzeigen.

4. Vorrichtung nach einem der Ansprüche 1-3, wobei:
die Vorrichtung zur Verwendung mit mindestens einem ersten und zweiten Werkzeug (21) dient;
die Robotereinheit (20) einen ersten Teil, der dazu konfiguriert ist, das erste Werkzeug über sechs Freiheitsgrade zu bewegen, und einen zweiten Teil umfasst, der dazu konfiguriert ist, das zweite Werkzeug über sechs Freiheitsgrade zu bewegen;
der mindestens eine Steuerkomponentenarm (70) zwei Steuerkomponentenarme (70) umfasst, wobei ein erster der Steuerkomponentenarme (70) dazu konfiguriert ist, von einer rechten Hand des Benutzers bewegt zu werden, und ein zweiter der Steuerkomponentenarme (70) dazu konfiguriert ist, von einer linken Hand des Benutzers bewegt zu werden, und
wobei der Computerprozessor (28) dazu konfiguriert ist, den ersten Teil der Robotereinheit (20) als Reaktion auf die Bewegung des ersten der Steuerkomponentenarme (70) zu steuern und den zweiten Teil der Robotereinheit (20) als Reaktion auf die Bewegung des zweiten der Steuerkomponentenarme (70) zu steuern.

5. Vorrichtung nach Anspruch 4, wobei der erste und zweite Steuerkomponentenarm (70) asymmetrisch in Bezug zueinander sind.

6. Vorrichtung nach einem der Ansprüche 1-3, ferner umfassend eine am Kopf montierte Anzeige (96), die dazu konfiguriert ist, dem Benutzer Echtzeitbilder des Teils des Körpers des Patienten und des einen oder der mehreren Werkzeuge (21) anzuzeigen.

7. Vorrichtung nach Anspruch 6, wobei die am Kopf montierte Anzeige (96) eine stereoskopische am Kopf montierte Anzeige umfasst, die dazu konfiguriert ist, dem Benutzer stereoskopische Echtzeitbilder des Teils des Körpers des Patienten und des einen oder der mehreren Werkzeuge (21) anzuzeigen.

8. Vorrichtung nach Anspruch 1, wobei die Vielzahl von Armmotoren (34) dazu konfiguriert ist, die Werkzeughalterung (30) entlang der X-, Y- und Z-Achse sowie durch Nick- und Gierwinkeldrehungen zu bewegen.

9. Vorrichtung nach Anspruch 1, wobei das eine oder die mehreren Werkzeuge (21) zwei oder mehr Werkzeuge umfassen, wobei jedes der zwei oder mehr Werkzeuge jeweilige Umfänge aufweist, die sich voneinander unterscheiden, und wobei die Werkzeughalterung (30) dazu konfiguriert ist, jedes der zwei oder mehr Werkzeuge darauf zu halten.

10. Vorrichtung nach Anspruch 1, wobei der eine oder die mehreren Halterungsmotoren (36), die der Werkzeughalterung (30) zugeordnet sind, dazu konfiguriert sind, das Werkzeug (21) in Bezug auf die Werkzeughalterung (30) über einen weiteren Freiheitsgrad zu bewegen.

11. Vorrichtung nach Anspruch 1, wobei die Werkzeughalterung (30) dazu konfiguriert ist, das eine oder die mehreren Werkzeuge (21) sicher darauf zu halten, während ermöglicht wird, dass das eine oder die mehreren Werkzeuge (21) in Bezug auf die Werkzeughalterung (30) gerollt werden.

12. Vorrichtung zum Durchführen eines Eingriffs an einem Teil eines Körpers eines Patienten unter Verwendung eines oder mehrerer Werkzeuge (21), wobei die Vorrichtung umfasst:
eine Robotereinheit (20), die dazu konfiguriert ist, das Werkzeug über sechs Freiheitsgrade zu bewegen; und
eine Steuerkomponente (26), die mindestens einen Steuerkomponentenarm (70) umfasst, der dazu konfiguriert ist, von einem Benutzer bewegt zu werden, wobei der Steuerkomponentenarm (70) umfasst:
ein Steuerkomponentenwerkzeug (71), das am Ende des Steuerkomponentenarms (70) angeordnet ist, wobei das Steuerkomponentenwerkzeug (71) eine Spitze definiert;
drei Gelenke;
drei Drehgeber (74), wobei jeder der drei Drehgeber mit einem jeweiligen der Gelenke gekoppelt ist und dazu konfiguriert ist, die Bewegung des jeweiligen Gelenks zu erkennen und Drehgeberdaten, die für eine XYZ-Position der Spitze des Steuerkomponentenwerkzeugs (71) bezeichnend sind, als Reaktion darauf zu generieren; und
eine Trägheitsmesseinheit (76), die mindestens einen von einem Sensor umfasst, der ausgewählt ist aus der Gruppe bestehend aus: einem dreiachsigen Beschleunigungsmesser, einem dreiachsigen Gyroskop und einem dreiachsigen Magnetometer,
wobei die Trägheitsmesseinheit (76) dazu konfiguriert ist, Trägheitsmesseinheitsdaten zu generieren; und
einen Computerprozessor (28), der konfiguriert ist zum:
Empfangen der Drehgeberdaten und der Trägheitsmesseinheitsdaten,
Bestimmen, auf der Grundlage der Drehgeberdaten, der XYZ-Position der Spitze des Steuerkomponentenwerkzeugs (71) und
Bewegen der Robotereinheit (20),
**dadurch gekennzeichnet, dass**:
die Trägheitsmesseinheit (76) am Steuerkomponentenwerkzeug (71) angeordnet ist;
die von der Trägheitsmesseinheit (76) generierten Trägheitsmesseinheitsdaten für eine Ausrichtung der Spitze des Steuerkomponentenwerkzeugs (71) bezeichnend sind;
der Computerprozessor (28) ferner konfiguriert ist zum:
Bestimmen der Ausrichtung der Spitze des Steuerkomponentenwerkzeugs (71) auf der Grundlage der Trägheitsmesseinheitsdaten, und
Bewegen der Robotereinheit (20) als Reaktion auf die XYZ-Position und die Ausrichtung der Spitze des Steuerkomponentenwerkzeugs (71), die vom Computerprozessor (28) bestimmt werden;
die Robotereinheit (20) dazu konfiguriert ist, unter Verwendung des einen oder der mehreren Werkzeuge (21) eine intraokulare Operation an einem Auge des Patienten durchzuführen, und das eine oder die mehreren Werkzeuge (21) Spitzen beinhalten;
die Robotereinheit (20) eine Werkzeughalterung (30), die dazu konfiguriert ist, das eine oder die mehreren Werkzeuge (21) sicher darauf zu halten, und einen oder mehrere mehrgelenkige Arme (32) umfasst, die auf einer einzigen Seite der Werkzeughalterung (30) angeordnet sind und dazu konfiguriert sind, die Werkzeughalterung (30) beweglich zu stützen; und
der Computerprozessor (28) dazu konfiguriert ist, die Robotereinheit anzutreiben, um zumindest einen Teil eines Eingriffs am Auge des Patienten durch Bewegen der Spitze des Werkzeugs (21) in einer
gewünschten Art und Weise in Bezug auf das Auge durchzuführen, etwa um den Teil des Eingriffs durchzuführen, während der Eintritt des Werkzeugs (21) in das Auge des Patienten am Einschnittpunkt fixiert gehalten wird.

13. Vorrichtung nach Anspruch 12, wobei der eine oder die mehreren mehrgelenkigen Arme (32) dazu konfiguriert sind, die Werkzeughalterung (30) entlang der X-, Y- und Z-Achse sowie durch Nick- und Gierwinkeldrehungen zu bewegen, und wobei der Computerprozessor (28) dazu konfiguriert ist, den einen oder die mehreren mehrgelenkigen Arme (32) anzutreiben, um die Werkzeughalterung entlang der X-, Y- und Z-Achse sowie durch Nick- und Gierwinkeldrehungen zu bewegen, während der Eintritt des einen oder der mehreren Werkzeuge (21) in das Auge des Patienten am Einschnittpunkt fixiert gehalten wird.

14. Vorrichtung nach Anspruch 12, wobei der eine oder die mehreren mehrgelenkigen Arme (32) dazu konfiguriert sind, die Werkzeughalterung (30) entlang der X-, Y- und Z-Achse sowie durch Nick- und Gierwinkeldrehungen zu bewegen, und wobei der Computerprozessor (28) dazu konfiguriert ist, das Werkzeug (21) zum Rollen in Bezug auf die Werkzeughalterung (30) anzutreiben, während der Eintritt des Werkzeugs (21) in das Auge des Patienten am Einschnittpunkt fixiert gehalten wird.

## Revendications

1. Appareil permettant d'effectuer une procédure sur une partie du corps d'un patient à l'aide d'un ou plusieurs outils (21), l'appareil comprenant :
une unité robotique (20) conçue pour déplacer l'outil suivant six degrés de liberté ; et
un composant de commande (26) qui comprend au moins un bras de composant de commande (70) conçu pour être déplacé par un utilisateur, le bras de composant de commande (70) comprenant :
un outil de composant de commande (71) disposé au niveau de l'extrémité du bras de composant de commande (70), l'outil de composant de commande (71) définissant une pointe ;
trois articulations ;
trois codeurs rotatifs (74), chacun des trois codeurs rotatifs étant couplé à l'une respective des articulations et conçu pour détecter le déplacement de l'articulation respective et pour générer des données de codeur rotatif indiquant l'emplacement XYZ de la pointe de l'outil de composant de commande (71), en réponse à celui-ci ; et
une unité de mesure inertielle (76) comprenant au moins un capteur sélectionné dans le groupe constitué par : un accéléromètre à trois axes, un gyroscope à trois axes et un magnétomètre à trois axes,
l'unité de mesure inertielle (76) étant configurée pour générer des données d'unité de mesure inertielle ; et
un processeur informatique (28) configuré pour :
recevoir les données de codeur rotatif et les données d'unité de mesure inertielle,
sur la base des données de codeur rotatif, déterminer l'emplacement XYZ de la pointe de l'outil de composant de commande (71), et
déplacer l'unité robotique (20),
**caractérisé en ce que** :
l'unité de mesure inertielle (76) est disposée sur l'outil de composant de commande (71) ;
les données d'unité de mesure inertielle générées par l'unité de mesure inertielle (76) indiquent l'orientation de la pointe de l'outil de composant de commande (71) ;
le processeur informatique (28) est en outre configuré pour :
déterminer l'orientation de la pointe de l'outil de composant de commande (71) sur la base des données de l'unité de mesure inertielle, et
déplacer l'unité robotique (20) en réponse à l'emplacement XYZ et à l'orientation de la pointe de l'outil de composant de commande (71) déterminés par le processeur informatique (28) ; et
l'unité robotique comprend :
un support d'outil (30) conçu pour maintenir fermement lesdits un ou plusieurs outils (21) sur lui ;
au moins un bras à articulations multiples (32) disposé sur un côté du support d'outil (30) et conçu pour supporter de manière mobile le support d'outil (30) ;
une pluralité de bras moteurs (34) associés audit au moins un bras à articulations multiples (32), la pluralité de bras moteurs (34) étant conçus pour déplacer le support d'outil (30) suivant au moins cinq degrés de liberté ; et
un ou plusieurs supports moteurs (36) associés au support d'outil (30) et conçus pour déplacer l'outil (21) par rapport au support d'outil (30) suivant un sixième degré de liberté.

2. Appareil selon la revendication 1, ledit composant de commande (26) comprenant un ou plusieurs éléments de support de poignet (90) conçus pour soutenir un poignet de l'utilisateur pendant le déplacement du bras de composant de commande (70).

3. Appareil permettant d'effectuer une procédure sur une partie du corps d'un patient à l'aide d'un ou plusieurs outils (21), l'appareil comprenant :
une unité robotique (20) conçue pour déplacer l'outil suivant six degrés de liberté ; et
un composant de commande (26) qui comprend au moins un bras de composant de commande (70) conçu pour être déplacé par un utilisateur, le bras de composant de commande (70) comprenant :
un outil de composant de commande (71) disposé au niveau de l'extrémité du bras de composant de commande (70), l'outil de composant de commande (71) définissant une pointe ;
trois articulations ;
trois codeurs rotatifs (74), chacun des trois codeurs rotatifs étant couplé à l'une respective des articulations et conçu pour détecter le déplacement de l'articulation respective et pour générer des données de codeur rotatif indiquant l'emplacement XYZ de la pointe de l'outil de composant de commande (71), en réponse à celui-ci ; et
une unité de mesure inertielle (76) comprenant au moins un capteur sélectionné dans le groupe constitué par : un accéléromètre à trois axes, un gyroscope à trois axes et un magnétomètre à trois axes,
l'unité de mesure inertielle (76) étant configurée pour générer des données d'unité de mesure inertielle ; et
un processeur informatique (28) configuré pour :
recevoir les données de codeur rotatif et les données d'unité de mesure inertielle,
sur la base des données de codeur rotatif, déterminer l'emplacement XYZ de la pointe de l'outil de composant de commande (71), et
déplacer l'unité robotique (20),
**caractérisé en ce que** :
l'unité de mesure inertielle (76) est disposée sur l'outil de composant de commande (71) ;
les données d'unité de mesure inertielle générées par l'unité de mesure inertielle (76) indiquent l'orientation de la pointe de l'outil de composant de commande (71) ;
le processeur informatique (28) est en outre configuré pour :
déterminer l'orientation de la pointe de l'outil de composant de commande (71) sur la base des données de l'unité de mesure inertielle, et
déplacer l'unité robotique (20) en réponse à l'emplacement XYZ et à l'orientation de la pointe de l'outil de composant de commande (71) déterminés par le processeur informatique (28) ; et
le composant de commande comprend un affichage stéréoscopique intégré (94) qui est configuré pour afficher des images stéréoscopiques en temps réel de la partie du corps du patient et desdits un ou plusieurs outils (21) à l'utilisateur.

4. Appareil selon l'une quelconque des revendications 1 à 3 :
ledit appareil étant destiné à être utilisé avec au moins des premier et second outils (21) ;
ladite unité robotique (20) comprenant une première partie conçue pour déplacer le premier outil suivant six degrés de liberté, et une seconde partie conçue pour déplacer le second outil suivant six degrés de liberté ;
ledit au moins un bras de composant de commande (70) comprenant deux bras de composant de commande (70), le premier des bras de composant de commande (70) étant conçu pour être déplacé par la main droite de l'utilisateur et le second des bras de composant de commande (70) étant conçu pour être déplacé par la main gauche de l'utilisateur, et
ledit processeur informatique (28) étant conçu pour commander la première partie de l'unité robotique (20) en réponse au déplacement du premier des bras de composant de commande (70), et pour commander la seconde partie de l'unité robotique (20) en réponse au déplacement du second des bras de composant de commande (70).

5. Appareil selon la revendication 4, lesdits premier et second bras de composant de commande (70) étant asymétriques l'un par rapport à l'autre.

6. Appareil selon l'une quelconque des revendications 1 à 3, comprenant en outre un visiocasque (96) qui est configuré pour afficher des images en temps réel de la partie du corps du patient et desdits un ou plusieurs outils (21) à l'utilisateur.

7. Appareil selon la revendication 6, ledit visiocasque (96) comprenant un visiocasque stéréoscopique qui est configuré pour afficher des images stéréoscopiques en temps réel de la partie du corps du patient et lesdits un ou plusieurs outils (21) à l'utilisateur.

8. Appareil selon la revendication 1, ladite pluralité de bras moteurs (34) étant conçus pour déplacer le support d'outil (30) le long des axes x, y et z, ainsi que par des rotations angulaires de tangage et de lacet.

9. Appareil selon la revendication 1, lesdits un ou plusieurs outils (21) comprenant deux outils ou plus, chacun des deux outils ou plus comportant des circonférences respectives qui diffèrent les unes des autres, et ledit support d'outil (30) étant conçu pour maintenir fermement chacun des deux outils ou plus sur lui.

10. Appareil selon la revendication 1, lesdits un ou plusieurs supports moteurs (36) associés au support d'outil (30) étant conçus pour déplacer l'outil (21) par rapport au support d'outil (30) suivant un degré de liberté supplémentaire.

11. Appareil selon la revendication 1, ledit support d'outil (30) étant conçu pour maintenir fermement lesdits un ou plusieurs outils (21) sur lui, tout en permettant auxdits un ou plusieurs outils (21) d'être roulés par rapport au support d'outil (30).

12. Appareil permettant d'effectuer une procédure sur une partie du corps d'un patient à l'aide d'un ou plusieurs outils (21), l'appareil comprenant :
une unité robotique (20) conçue pour déplacer l'outil suivant six degrés de liberté ; et
un composant de commande (26) qui comprend au moins un bras de composant de commande (70) conçu pour être déplacé par un utilisateur, le bras de composant de commande (70) comprenant :
un outil de composant de commande (71) disposé au niveau de l'extrémité du bras de composant de commande (70), l'outil de composant de commande (71) définissant une pointe ;
trois articulations ;
trois codeurs rotatifs (74), chacun des trois codeurs rotatifs étant couplé à l'une respective des articulations et conçu pour détecter le déplacement de l'articulation respective et pour générer des données de codeur rotatif indiquant l'emplacement XYZ de la pointe de l'outil de composant de commande (71), en réponse à celui-ci ; et
une unité de mesure inertielle (76) comprenant au moins un capteur sélectionné dans le groupe constitué par : un accéléromètre à trois axes, un gyroscope à trois axes et un magnétomètre à trois axes,
l'unité de mesure inertielle (76) étant configurée pour générer des données d'unité de mesure inertielle ; et
un processeur informatique (28) configuré pour :
recevoir les données de codeur rotatif et les données d'unité de mesure inertielle,
sur la base des données de codeur rotatif, déterminer l'emplacement XYZ de la pointe de l'outil de composant de commande (71), et
déplacer l'unité robotique (20),
**caractérisé en ce que** :
l'unité de mesure inertielle (76) est disposée sur l'outil de composant de commande (71) ;
les données d'unité de mesure inertielle générées par l'unité de mesure inertielle (76) indiquent l'orientation de la pointe de l'outil de composant de commande (71) ;
le processeur informatique (28) est en outre configuré pour :
déterminer l'orientation de la pointe de l'outil de composant de commande (71) sur la base des données de l'unité de mesure inertielle, et
déplacer l'unité robotique (20) en réponse à l'emplacement XYZ et à l'orientation de la pointe de l'outil de composant de commande (71) déterminés par le processeur informatique (28) ;
l'unité robotique (20) est conçue pour effectuer une chirurgie intraoculaire sur un oeil du patient à l'aide desdits un ou plusieurs outils (21), et lesdits un ou plusieurs outils (21) comprennent des pointes ;
l'unité robotique (20) comprend un support d'outil (30) conçu pour maintenir fermement lesdits un ou plusieurs outils (21) sur lui, et un ou plusieurs bras à articulations multiples (32) disposés sur un seul côté du support d'outil (30) et conçu pour soutenir de manière mobile le support d'outil (30) ; et
le processeur informatique (28) est configuré pour actionner l'unité robotique de manière à effectuer au moins une partie d'une procédure sur l'œil du patient en déplaçant la pointe de l'outil (21) de manière souhaitée par rapport à l'oeil de manière à effectuer la partie de la procédure, tandis que l'entrée de l'outil (21) dans l'oeil du patient est maintenue fixe au niveau du point d'incision.

13. Appareil selon la revendication 12, lesdits un ou plusieurs bras à articulations multiples (32) étant conçus pour déplacer le support d'outil (30) le long des axes x, y et z, ainsi que par des rotations angulaires de tangage et de lacet, et ledit processeur informatique (28) étant configuré pour actionner lesdits un ou plusieurs bras à articulations multiples (32) de manière à déplacer le support d'outil le long des axes x, y et z, ainsi que par des rotations angulaires de tangage et de lacet, tandis que l'entrée desdits un ou plusieurs outils (21) dans l'oeil du patient est maintenue fixe au niveau du point d'incision.

14. Appareil selon la revendication 12, lesdits un ou plusieurs bras à articulations multiples (32) étant conçus pour déplacer le support d'outil (30) le long des axes x, y et z, ainsi que par des rotations angulaires de tangage et de lacet, et ledit processeur informatique (28) étant configuré pour actionner l'outil (21) de manière à rouler par rapport au support d'outil (30), tandis que l'entrée de l'outil (21) dans l'oeil du patient est maintenue fixe au niveau du point d'incision.
